# EUROPEAN PATENT APPLICATION

(11) **EP 4 324 909 A1**
(43) Date of publication of application: **21.02.2024**
(21) Application number: 22788418.6
(22) Date of filing: 12.04.2022
(51) Int. Cl.: C12M 3/00, C12N 5/071, C12N 5/077, G01N 33/50

(54) **KIDNEY-ON-A-CHIP COMPRISING RENAL TUBULE CULTURE PART AND INTERSTITIUM CULTURE PART**

(30) Priority: 12.04.2021 KR 20210047427
(71) Applicant: Seoul National University Hospital, Seoul 03080 (KR); Seoul National University R&DB Foundation, Seoul 08826 (KR)
(72) Inventor: KIM, Sejoong, Seoul 06281 (KR); LEE, Yun-Mi, Seongnam-si, Gyeonggi-do 13589 (KR); HWANG, Seong-Hye, Seongnam-si, Gyeonggi-do 13607 (KR); JEON, Noo Li, Seoul 08826 (KR); LIM, Jungeun, Seoul 08826 (KR)
(74) Representative: V.O.
(86) International application number: PCT/KR2022/005315
(87) International publication number: WO 2022/220562

(57) **Abstract**

The present invention relates to: an organ-on-a-chip for mimicking kidney tissue in which renal tubular epithelial cells and renal fibroblasts are co-cultured; a method for manufacturing the organ-on-a-chip; a device for co-culturing three types of cells; and a method for screening for renal fibrosis therapeutic agents by using the organ-on-a-chip. In a kidney-on-a-chip, according to one aspect of the present invention, renal tubular epithelial cells and renal fibroblasts can be co-cultured by using a fibrin gel and not using a membrane, which has been conventionally used, without culture solution leakage and physical deformation and, additionally, vascular endothelial cells can be co-cultured together so that kidney tissue is more perfectly mimicked, the structure of the present invention is simplified into a renal tubule culture part and an interstitium culture part and is horizontally arranged, and thus, even without a separate culture solution feeding channel, drug administration to each compartment and intercellular substance exchange are easy, the efficiency of staining is increased, and the manufacturing period and cost of the organ-on-a-chip are reduced. Therefore, a kidney-on-a-chip according to one embodiment of the present invention has the excellent effects of enabling the time and errors required in screening for renal fibrosis therapeutic agents to be minimized and renal fibrosis therapeutic agents to be more conveniently and simply screened for through an objective evaluation index.

## Description

### [Technical Field]

This study has been conducted under the supervision of Seoul National University Bundang Hospital with support of the Ministry of Science and ICT for the mid-career researcher support project, and the name of this study is the development of a renal fibrosis model using organ-on-a-chip technique (project identification number: 1711085548). This study has also been conducted under the supervision of Seoul National University with support of the Ministry of Science and ICT for the personal basic research (MSIT) (R&D) project, and the name of this study is Eye-on-a-Chip: Development of multiplex eye mimicking platform for multiple stimulation and analysis (project identification number: 1711112005).

The present specification discloses an organ-on-a-chip for mimicking kidney tissue in which renal tubular epithelial cells and renal fibroblasts are co-cultured; a method for manufacturing the organ-on-a-chip; a device for co-culturing three types of cells; and a method for screening for renal fibrosis therapeutic agents using the organ-on-a-chip.

### [Background Art]

Due to an aging society and an increase in patients with diabetes and high blood pressure, the number of patients with chronic renal failure is rapidly increasing, and chronic renal failure becomes an incurable disease in which renal function cannot be improved except through dialysis or transplantation when it progresses to end-stage renal failure. The number of patients with end-stage renal failure has exceeded 70,000 in 2014, and medical insurance benefits related to this kidney disease are approaching KRW 2 trillion.

In comparison, there are no drug directly used to treat chronic renal failure, and the development of effective drugs for the treatment of kidney disease is still in its infancy. This is because there are limitations of high cost and low efficiency such as difficulties in recruiting patients for clinical trials and the need for long-term follow-up as well as the fact that an animal model of chronic renal failure that can represent the human model has not yet been established. Therefore, there is an urgent need to develop an appropriate renal failure model to reveal the pathophysiology of chronic kidney disease and to be used in the therapeutic application.

Unlike other organs, kidney tissue is not only structurally complex, but also various cells are exposed to a variety of variable microenvironments such as blood pressure and blood flow, so treatment of kidney disease requires a multifaceted approach. In particular, renal fibrosis is a common phenomenon seen in most kidney diseases that progress to chronic renal failure, and is closely related to inflammation and fibrosis of the renal tubular interstitium rather than glomerular lesions.

There are several emerging hypotheses, including the hypothesis that renal fibrosis is caused by fibroblasts proliferating through the epithelial mesenchymal transition (EMT) process and the hypothesis that renal fibrosis is caused by Wnt/Shh signaling in renal tubular epithelial cells and Shh signaling activating fibroblasts; however, an exact mechanisms regarding underlying cells of fibrosis or regulation of fibrosis are unknown.

Meanwhile, preclinical experiments consist of cell experiments (*in vitro*) and animal experiments. However, cell experiments are two dimensional and do not fully reflect the *in vivo* microenvironment, and animal experiments also have problems such as high cost, ethical issues, and limitations in direct application to humans due to differences between species. Because of these limitations, 40% of drugs that have passed preclinical trials are rejected in actual clinical trials due to toxicity and efficacy issues, resulting in a serious medical, economic, and industrial burden.

Organ-on-a-chip technology, which is emerging as a way to overcome this, is a technology for culturing cells in a chip that reproduces the *in vivo* microenvironment using microfluidic technology, and is in the spotlight as groundbreaking technology that can achieve synergy with the advantages of cell experiments and animal experiments and eliminate the disadvantages. In other words, it is possible to provide engineering tools at a low price by expanding existing cell culture models through organ-on-a-chip technology, and the organ-on-a-chip technology thus has the advantage of dramatically reducing the costs of existing animal experiments and clinical trials.

Existing animal models of renal fibrosis include unilateral ureteral obstruction or ischemia-reperfusion models and mildly toxic renal fibrosis models, which have been studied as preclinical trial models. However, these renal fibrosis models have limitations in reflecting renal fibrosis in the human body. In addition, renal tubular epithelial cells and renal fibroblasts are emerging as cells that play a major role in reproducing renal fibrosis, so it is essential to develop a model in which the two cells can be co-cultured.

Existing kidney organ-on-chips have been developed using only renal tubule cells and have limitations in revealing renal fibrosis, and there is a need to develop a systematic organ-on-a-chip through co-culture of renal tubule cells and renal fibroblasts.

Accordingly, the present inventors have conducted studies to develop an organ-on-a-chip in which renal tubular epithelial cells and renal fibroblasts can be co-cultured, and to develop an organ-on-a-chip that can mimic renal fibrosis based on this.

### [Summary of Invention]

### [Technical Problem]

In an aspect, an object of the present invention is to provide an organ-on-a-chip in which renal tubular epithelial cells and renal fibroblasts are co-cultured and which can mimic the kidney.

In another aspect, an object of the present invention is to provide a method for screening for renal fibrosis therapeutic agents using the organ-on-a-chip.

### [Solution to Problem]

In an aspect, the present invention provides a kidney-on-a-chip comprising a renal tubule culture part where renal tubular epithelial cells are cultured; and an interstitium culture part where renal fibroblasts and vascular endothelial cells are cultured, in which the renal tubule culture part and the interstitium culture part are horizontally arranged and the renal tubular epithelial cells, renal fibroblasts, and vascular endothelial cells are co-cultured.

In another aspect, the present invention provides a method for manufacturing the kidney-on-a-chip, the method comprising culturing renal tubular epithelial cells in the renal tubule culture part and culturing renal fibroblasts and vascular endothelial cells in the interstitium culture part.

In still another aspect, the present invention provides a co-culture device comprising a renal tubule culture part where renal tubular epithelial cells are cultured; and an interstitium culture part where renal fibroblasts and vascular endothelial cells are cultured, in which the renal tubule culture part and the interstitium culture part are horizontally arranged and the renal tubular epithelial cells, renal fibroblasts, and vascular endothelial cells are co-cultured.

In still another aspect, the present invention provides a method for screening for a renal fibrosis therapeutic agent, the method comprising: (a) treating the kidney-on-a-chip described above with transforming growth factor-β (TGF-β); (b) treating the kidney-on-a-chip with a test substance; and (c) measuring a renal fibrosis evaluation index in the kidney-on-a-chip treated with the test substance.

### [Advantageous Effects of Invention]

The kidney-on-a-chip according to an aspect of the present invention has an advantage of not leaking the culture solution and the like even without using a membrane, and more perfectly mimics kidney tissue since human kidney tissue-derived renal fibroblasts and renal tubular epithelial cells can be co-cultured in a three dimensional manner without physical deformation using blank fibrin gel and vascular epithelial cells can also be co-cultured together with these cells, the structure of the kidney-on-a-chip is simplified into a renal tubule culture part and an interstitium culture part and is horizontally arranged and thus drug administration to each compartment and intercellular substance exchange are easy even without a separate culture solution feeding channel, the efficiency of staining also is increased as well as the manufacturing period and cost of the organ-on-a-chip are reduced. Therefore, a kidney-on-a-chip according to an embodiment of the present invention has the excellent effects of enabling the time and errors required to screen for renal fibrosis therapeutic agents to be minimized and renal fibrosis therapeutic agents to be more conveniently and simply screened for through an objective evaluation index.

### [Brief Description of Drawings]

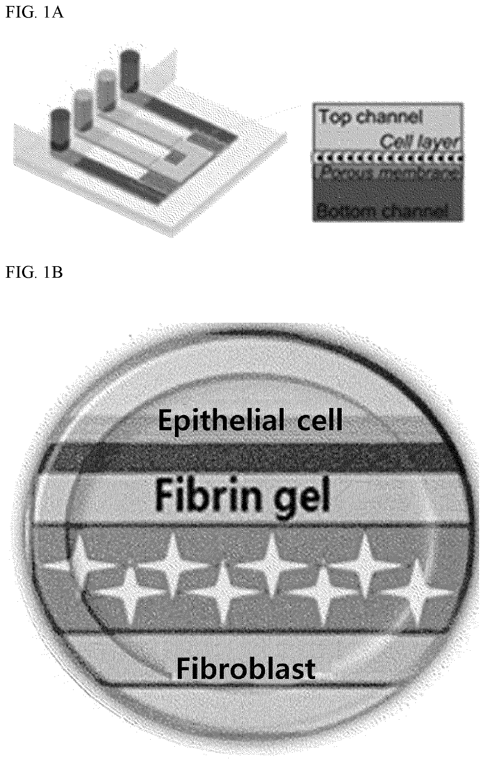

FIG. 1A is a schematic diagram of an existing kidney-on-a-chip having a vertical multilayer structure of upper and lower layers for co-culturing renal tubular epithelial cells and renal fibroblasts;
FIG. 1B is a schematic diagram of a kidney-on-a-chip having a horizontal multi-compartment structure for co-culturing rodent-derived renal tubular epithelial cells and renal fibroblasts according to an embodiment of the present invention;

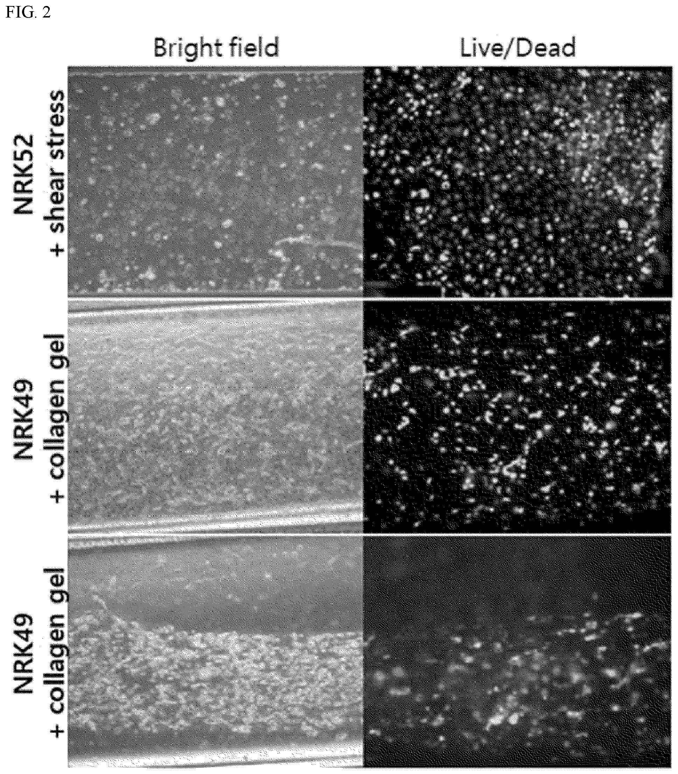

FIG. 2 is photographs illustrating the proliferation of each cell when renal tubular epithelial cells (NRK 52) and renal fibroblasts (NRK 49) are cultured in an existing kidney-on-a-chip manufactured using collagen gel;

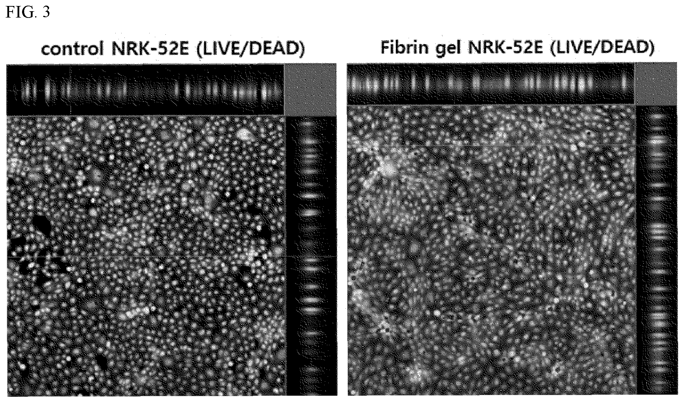

FIG. 3 is photographs illustrating the proliferation of each cell when rodent-derived renal tubular epithelial cells (NRK 52) according to an embodiment of the present invention are cultured in a general medium and when the cells are cultured on fibrin gel;

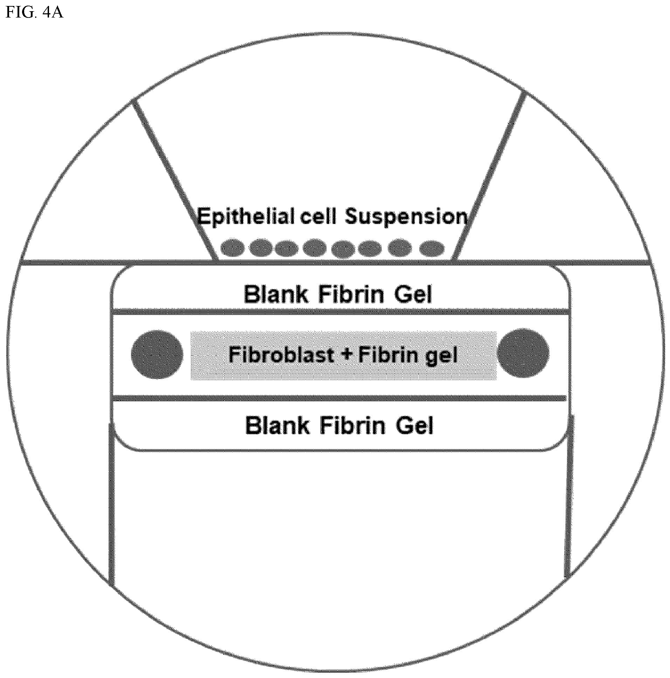

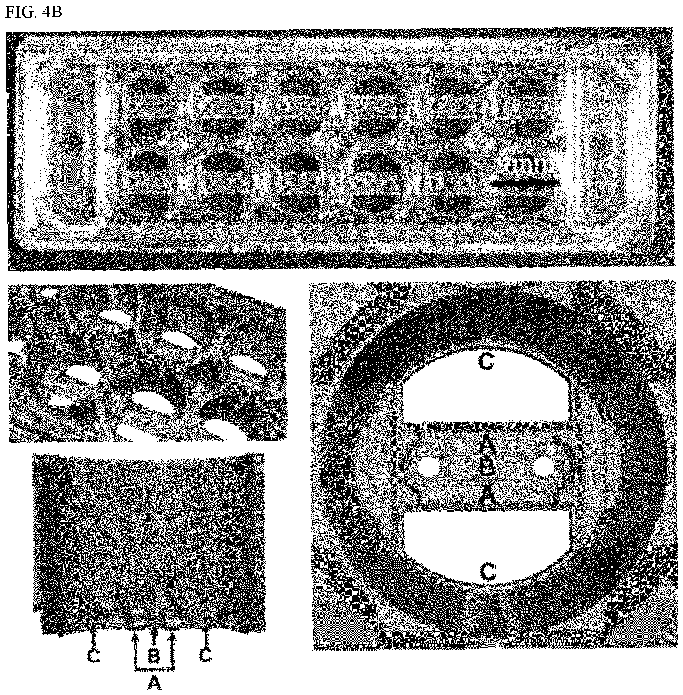

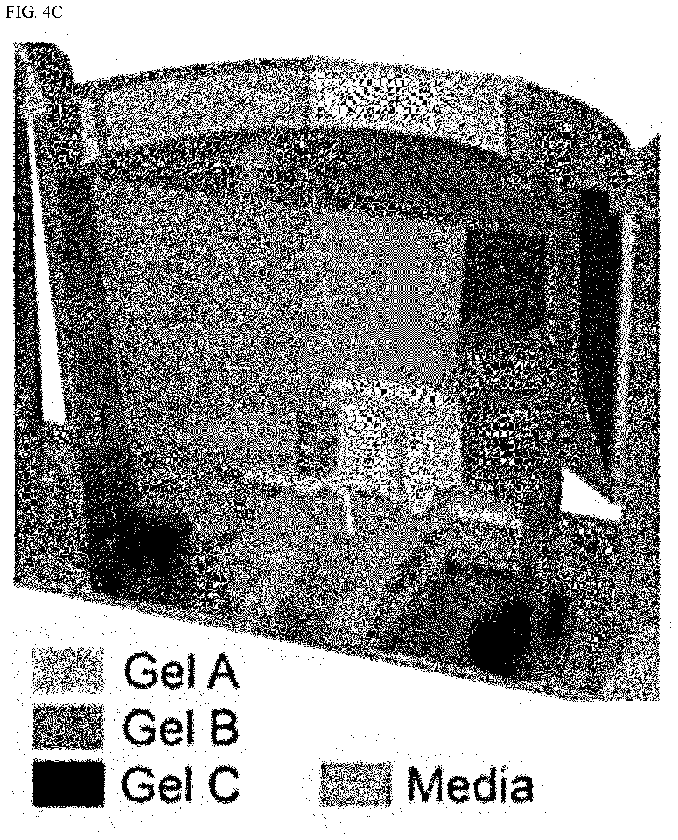

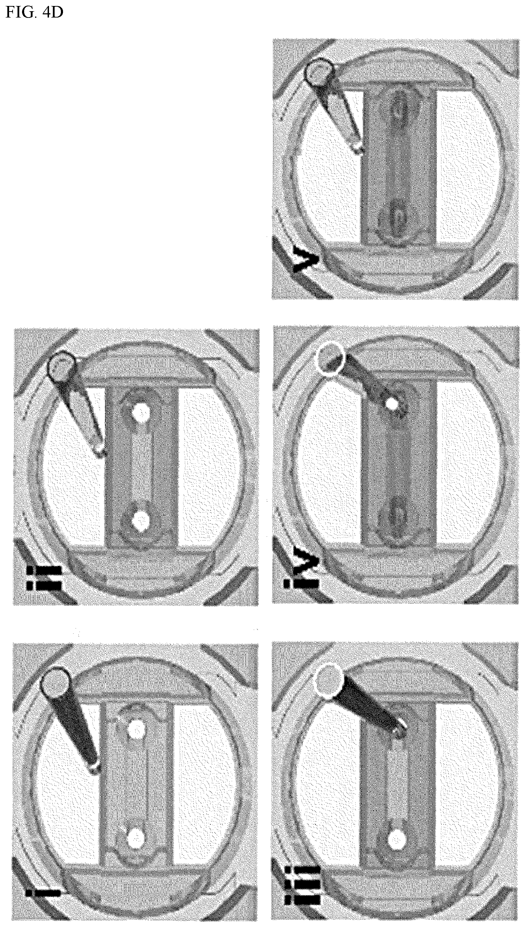

FIGS. 4A to 4D are a schematic diagram (FIG 4A) and photographs (FIG 4B) of a kidney-on-a-chip manufactured using rodent-derived renal tubular epithelial cells and renal fibroblasts according to an embodiment of the present invention, a schematic diagram (FIG. 4C) illustrating each region of the kidney-on-a-chip, and diagrams (FIG. 4D) illustrating the process of seeding cells and the like in each region;

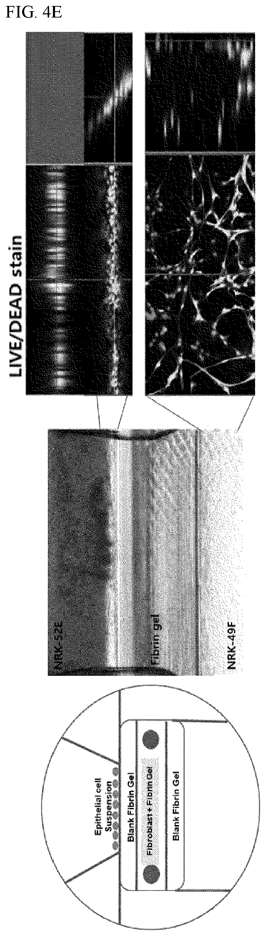

FIG. 4E is a schematic diagram (left) and a photograph (middle) of a kidney-on-a-chip manufactured using rodent-derived renal tubular epithelial cells and renal fibroblasts according to an embodiment of the present invention, and photographs (right) illustrating the results of staining the renal tubular epithelial cells and renal fibroblasts;

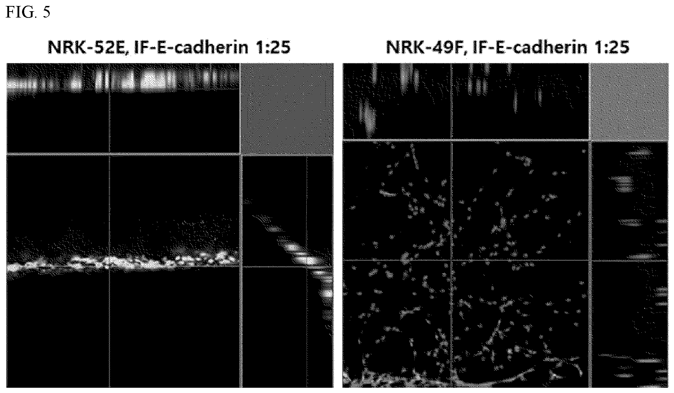

FIG. 5 is photographs illustrating E-cadherin fluorescence expression in each of the renal tubular epithelial cells (NRK 52) (left) and renal fibroblasts (NRK 49) (right) in a kidney-on-a-chip manufactured using rodent-derived renal tubular epithelial cells and renal fibroblasts according to an embodiment of the present invention;

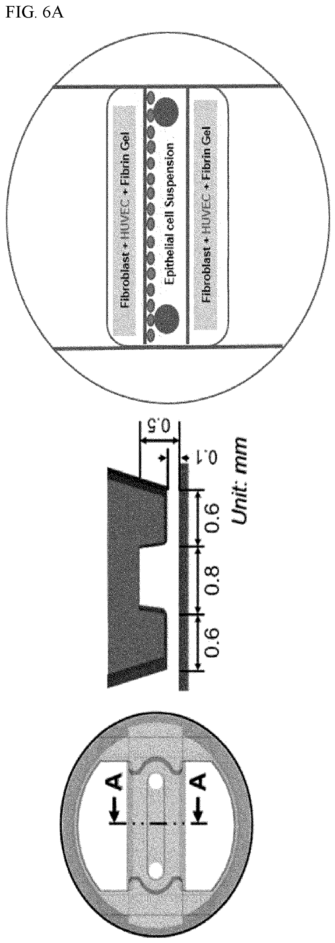

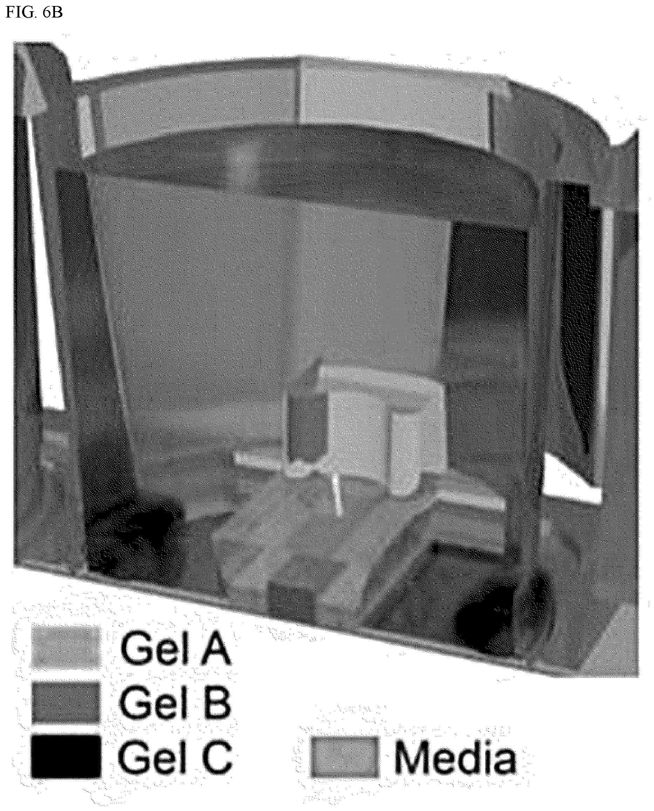

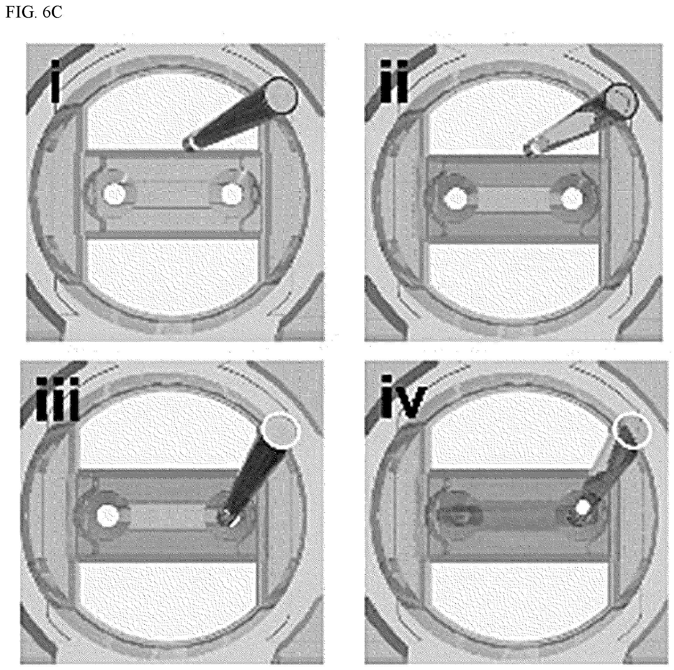

FIGS. 6A to 6C are schematic diagrams (FIG 6A) of a kidney-on-a-chip that has a horizontal structure and is manufactured using rodent-derived renal tubular epithelial cells and renal fibroblasts according to an embodiment of the present invention, a schematic diagram (FIG. 6B) illustrating each region of the kidney-on-a-chip, and diagrams (FIG. 6C) illustrating the process of seeding cells and the like in each region;

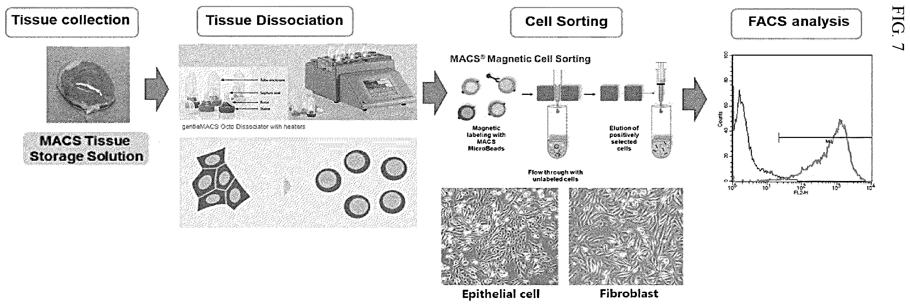

FIG. 7 is schematic diagrams illustrating a method for isolating renal tubular epithelial cells and renal fibroblasts from human kidney tissue according to an embodiment of the present invention;

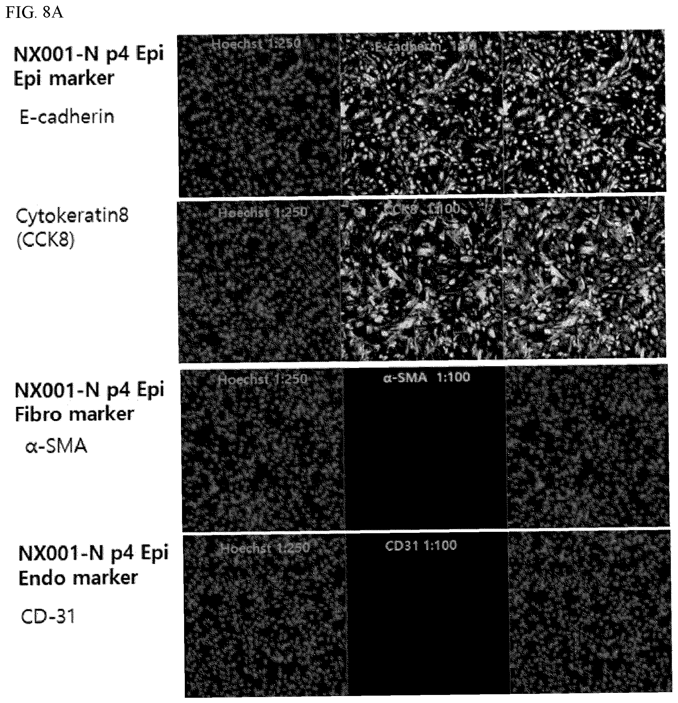

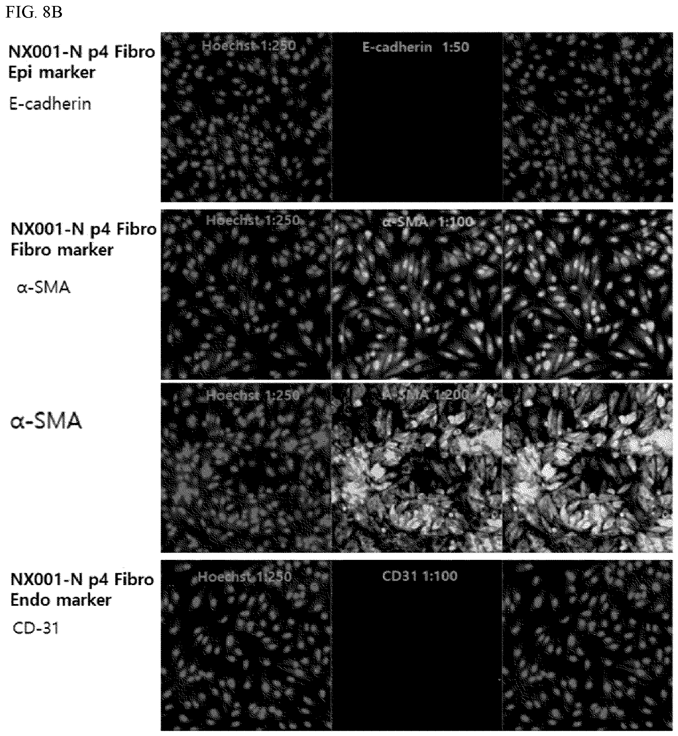

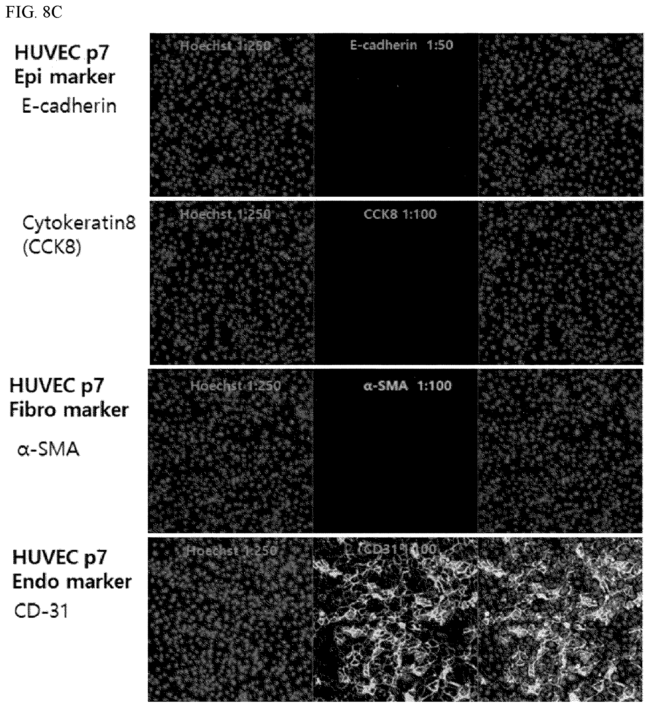

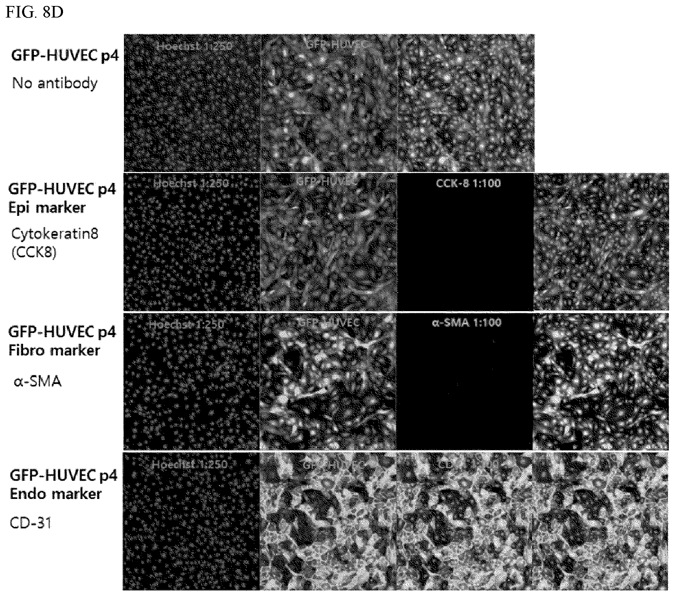

FIGS. 8A to 8D are photographs illustrating fluorescence expression of E-cadherin, cytokeratin 8 (CCK8), α-smooth muscle actin (α-SMA), and cluster of differentiation 31 (CD31) in each of renal tubular epithelial cells (Epi) (FIG. 8A) and renal fibroblasts (Fibro) (FIG. 8B) isolated from human kidney tissue by a method according to an embodiment of the present invention, and vascular endothelial cells (HUVEC, GFP-HUVEC) (FIGS. 8C and 8D);

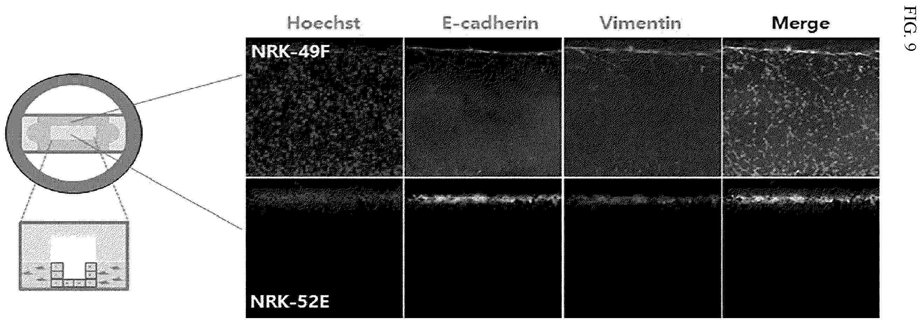

FIG. 9 is a schematic diagram (left) of a kidney-on-a-chip manufactured using rodent-derived renal tubular epithelial cells and renal fibroblasts according to an embodiment of the present invention, and photographs (right) illustrating fluorescence expression of E-cadherin and vimentin in the renal tubular epithelial cells (NRK 52E) and renal fibroblasts (NRK 49F);

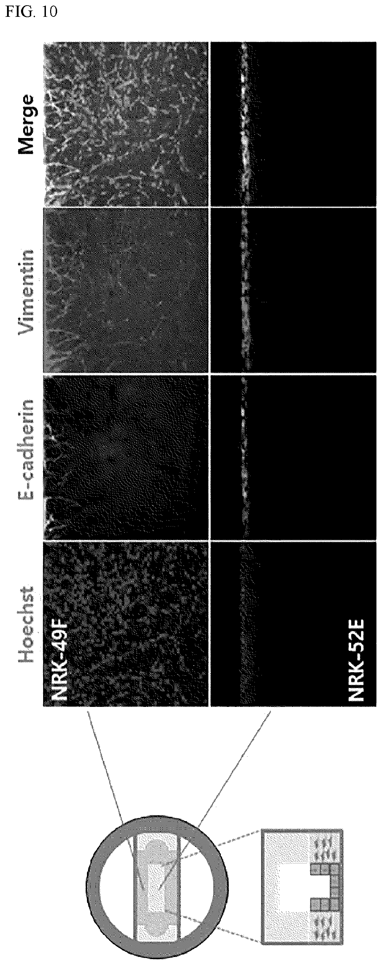

FIG. 10 is a schematic diagram (left) of a kidney-on-a-chip manufactured using rodent-derived renal tubular epithelial cells and renal fibroblasts at the time of TGF-β stimulation according to an embodiment of the present invention, and photographs (right) illustrating fluorescence expression of E-cadherin and vimentin in the renal tubular epithelial cells (NRK 52E) and renal fibroblasts (NRK 49F);

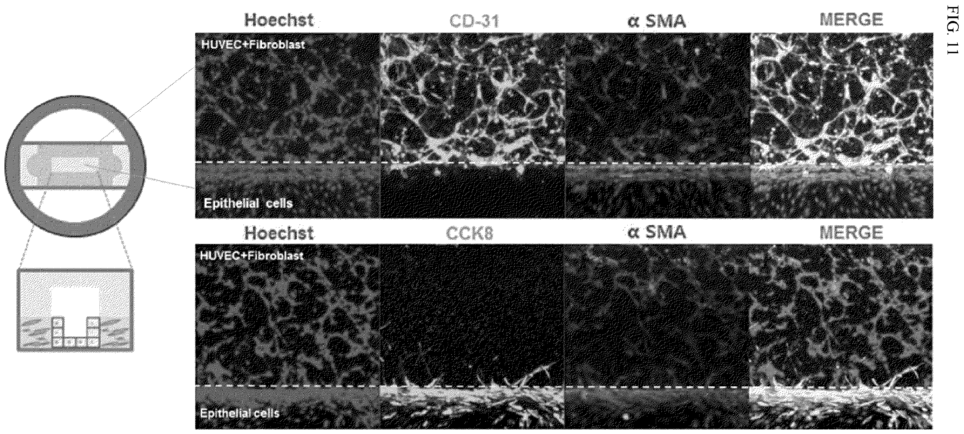

FIG. 11 is a schematic diagram (left) of a kidney-on-a-chip manufactured using human-derived renal tubular epithelial cells and renal fibroblasts at the time of TGF-β stimulation according to an embodiment of the present invention, and photographs (right) illustrating fluorescence expression of CCK8, CD31, and α-SMA in each of the renal tubular epithelial cell compartment (renal tubule culture part) and the interstitium compartment (interstitium culture part);

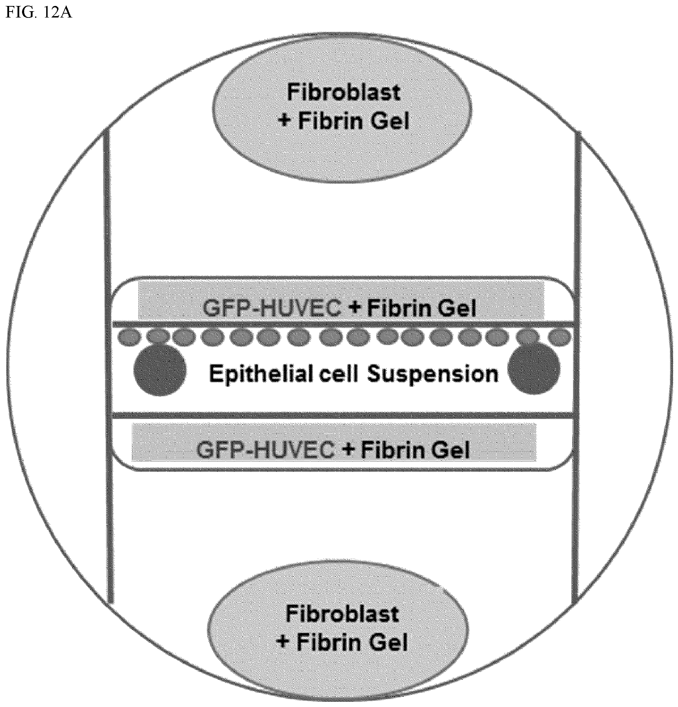

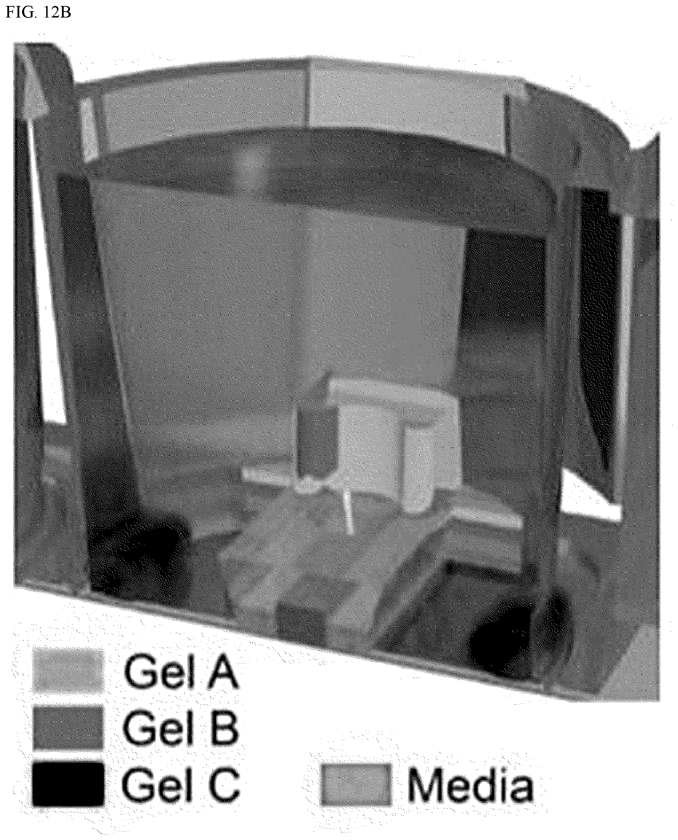

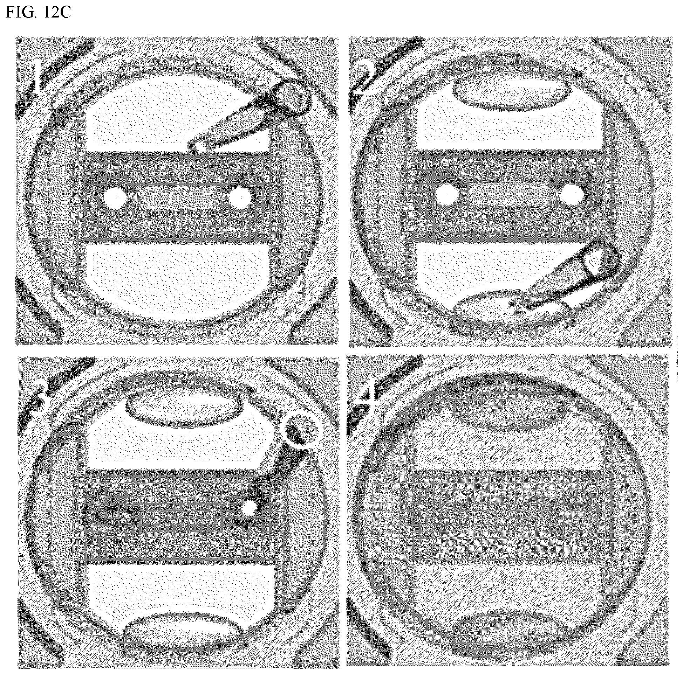

FIGS. 12A to 12C are schematic diagrams (FIG 12A) of a renal fibrosis-on-a-chip manufactured using renal fibrosis tissue-derived renal tubular epithelial cells and renal fibroblasts according to an embodiment of the present invention, a schematic diagram (FIG. 12B) illustrating each region of the kidney-on-a-chip, and diagrams (FIG. 12C) illustrating the process of seeding cells and the like in each region;

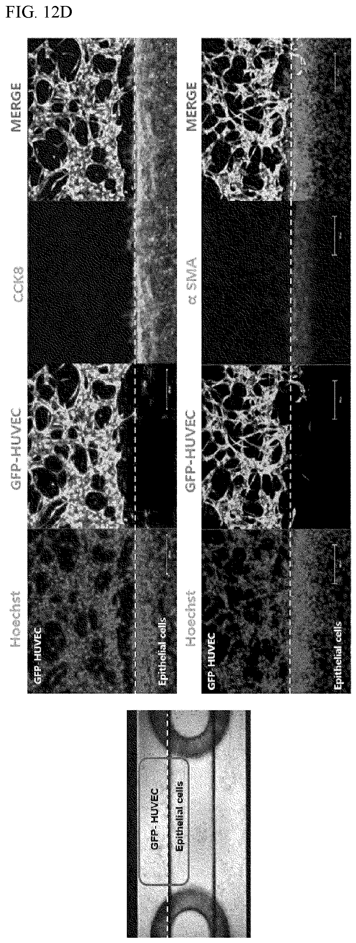

FIG. 12D is an actual photograph (left) of a renal fibrosis-on-a-chip manufactured using renal fibrosis tissue-derived renal tubular epithelial cells and renal fibroblasts according to an embodiment of the present invention, and photographs (right) illustrating fluorescence expression of vascular endothelial cells (GFP-HUVEC), CCK8, and α-SMA in each of the renal tubular epithelial cell compartment (renal tubule culture part) and the interstitium compartment (interstitium culture part);

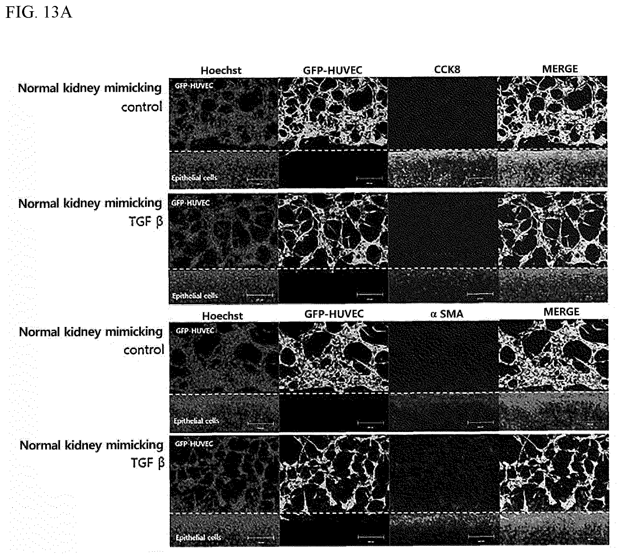

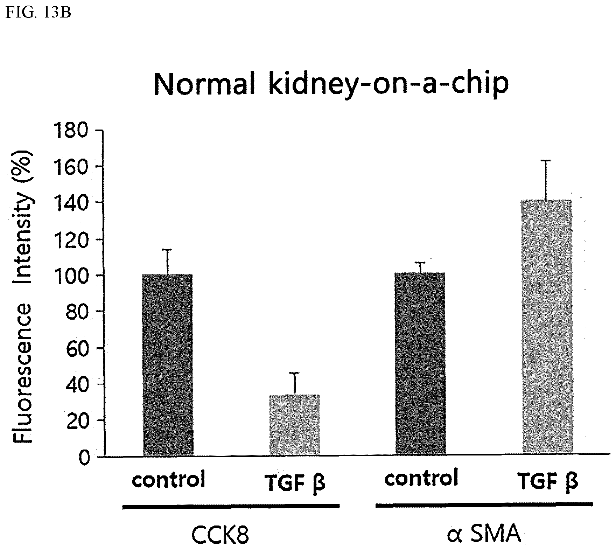

FIGS. 13A and 13B are photographs (FIG 13A) illustrating fluorescence expression of vascular endothelial cells (GFP-HUVEC), CCK8, and α-SMA in kidney-on-chips manufactured using renal tubular epithelial cells and normal kidney tissue-derived renal fibroblasts according to an embodiment of the present invention and in the kidney-on-chips at the time of TGF-β stimulation, and a graph (FIG. 13B) illustrating quantified expression levels of CCK8 and α-SMA;

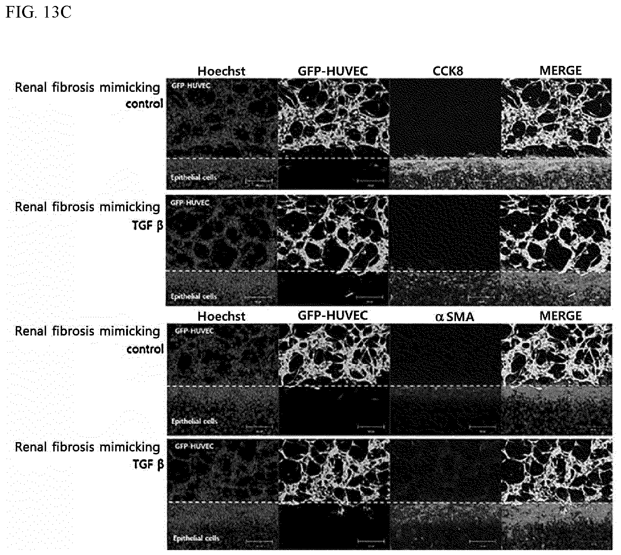

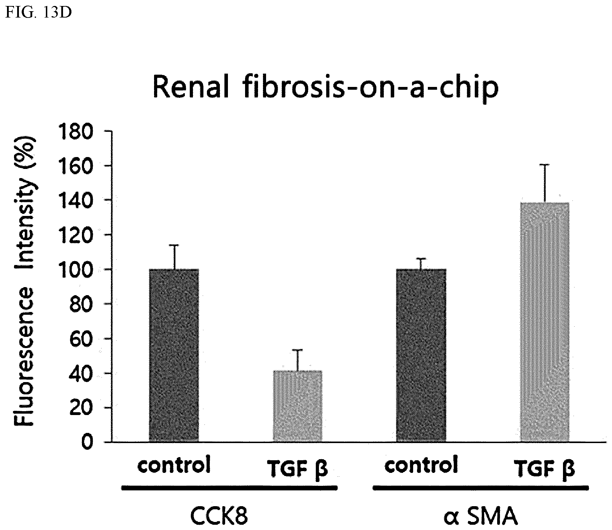

FIGS. 13C and 13D are photographs (FIG 13C) illustrating fluorescence expression of vascular endothelial cells (GFP-HUVEC), CCK8, and α-SMA in renal fibrosis-on-chips manufactured using renal tubular epithelial cells and renal fibrosis tissue-derived fibroblasts according to an embodiment of the present invention and in the renal fibrosis-on-chips at the time of TGF-β stimulation, and a graph (FIG. 13D) illustrating quantified expression levels of CCK8 and α-SMA;

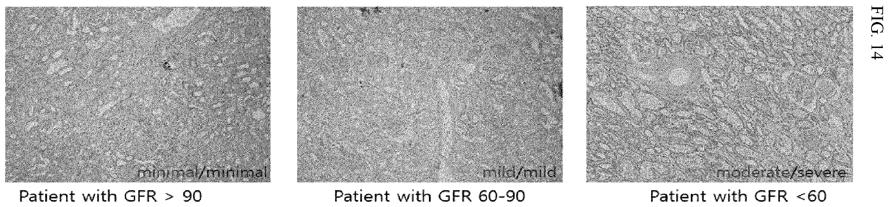

FIG. 14 is photographs illustrating the degree of damage to kidney tissue in each of three patients with renal fibrosis according to an embodiment of the present invention, in which red text indicates the degree of tubular atrophy and interstitial fibrosis and blue text indicates the degree of fibro-intimal thickening/arteriolosclerosis;

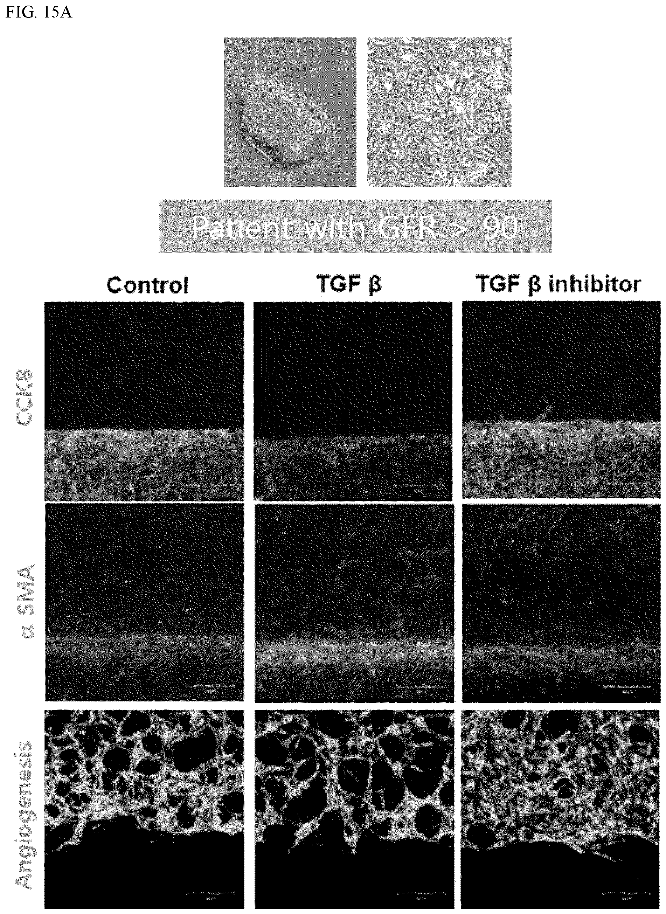

FIGS. 15A to 15C are photographs illustrating fluorescence expression of CCK8 and α-SMA, which are renal fibrosis evaluation indices, and photographs illustrating fluorescence expression (angiogenesis) of vascular endothelial cells to measure the degree of angiogenesis in renal fibrosis-on-chips manufactured using cells derived from each of three patients with renal fibrosis according to an embodiment of the present invention before TGF-β stimulation (control), after TGF-β stimulation (TGF-β), and after TGF-β inhibitor administration (TGF-β inhibitor);

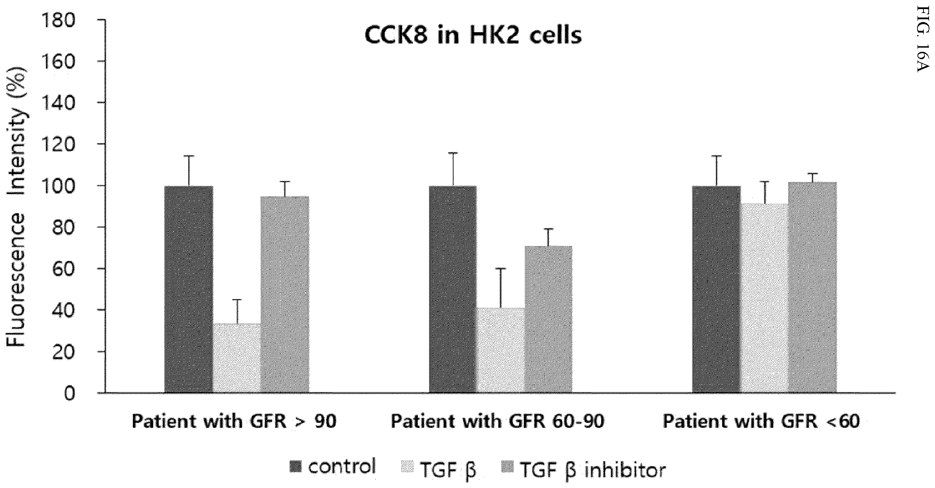

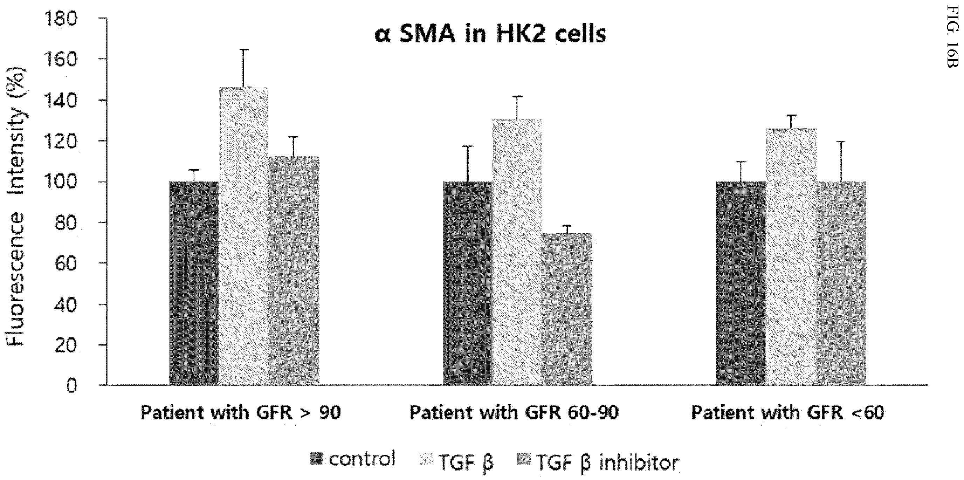

FIGS. 16A and 16B are graphs illustrating the expression levels of CCK8 (FIG. 16A) and α-SMA (FIG. 16B), which are renal fibrosis evaluation indices, in renal fibrosis-on-chips manufactured using cells derived from a patient with renal fibrosis having a GFR of more than 90, cells from a patient with renal fibrosis having a GFR of 60 to 90, and cells from a patient with renal fibrosis having a GFR of less than 60 according to an embodiment of the present invention before TGF-β stimulation (control), after TGF-β stimulation (TGF-β), and after TGF-β inhibitor administration (TGF-β inhibitor);

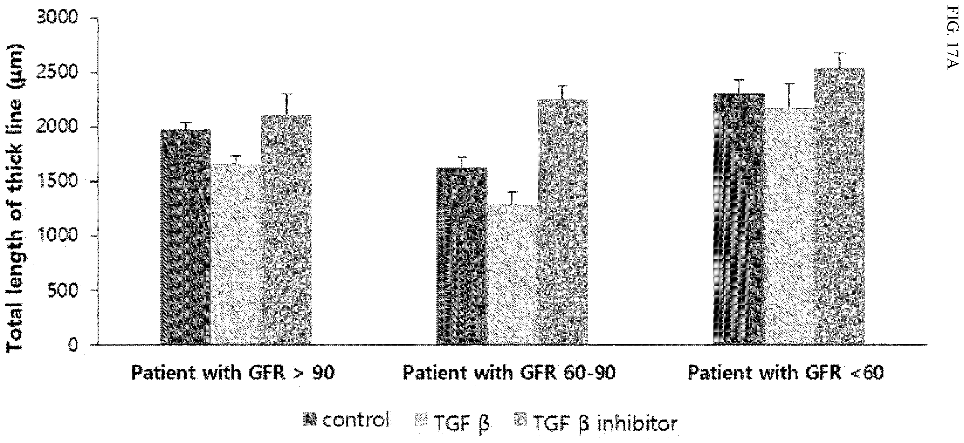

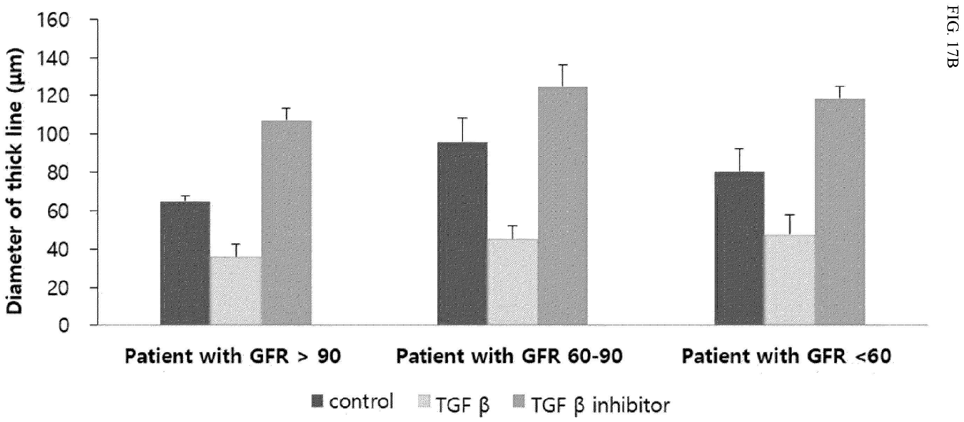

FIGS. 17A and 17B are graphs illustrating the sum (FIG. 17A) of lengths of thick blood vessels having a diameter of 50 µm or more and the average (FIG. 17B) of diameters of thick blood vessels of vascular endothelial cells to measure the degree of angiogenesis, which is a renal fibrosis evaluation index, in renal fibrosis-on-chips manufactured using cells derived from a patient with renal fibrosis having a GFR of more than 90, cells from a patient with renal fibrosis having a GFR of 60 to 90, and cells from a patient with renal fibrosis having a GFR of less than 60 according to an embodiment of the present invention before TGF-β stimulation (control), after TGF-β stimulation (TGF-β), and after TGF-β inhibitor administration (TGF-β inhibitor);

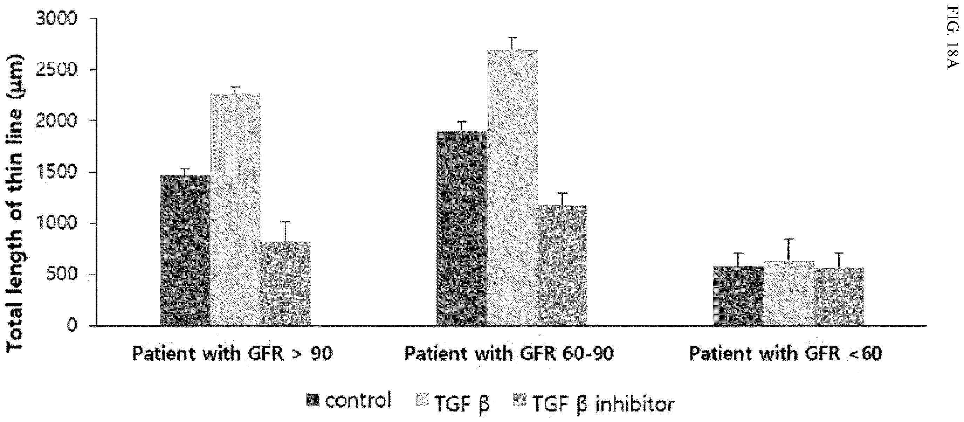

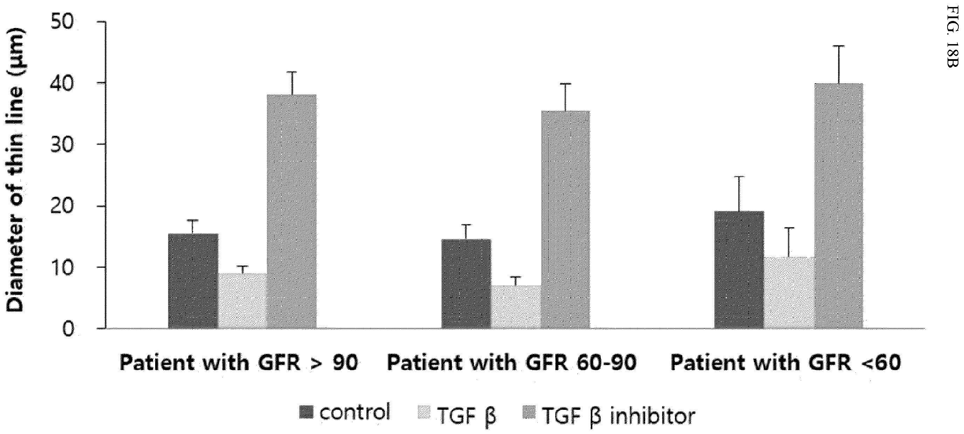

FIGS. 18A and 18B are graphs illustrating the sum (FIG. 18A) of lengths of thin blood vessels having a diameter of less than 50 µm and the average (FIG. 18B) of diameters of thin blood vessels of vascular endothelial cells to measure the degree of angiogenesis, which is a renal fibrosis evaluation index, in renal fibrosis-on-chips manufactured using cells derived from a patient with renal fibrosis having a GFR of more than 90, cells from a patient with renal fibrosis having a GFR of 60 to 90, and cells from a patient with renal fibrosis having a GFR of less than 60 according to an embodiment of the present invention before TGF-β stimulation (control), after TGF-β stimulation (TGF-β), and after TGF-β inhibitor administration (TGF-β inhibitor);

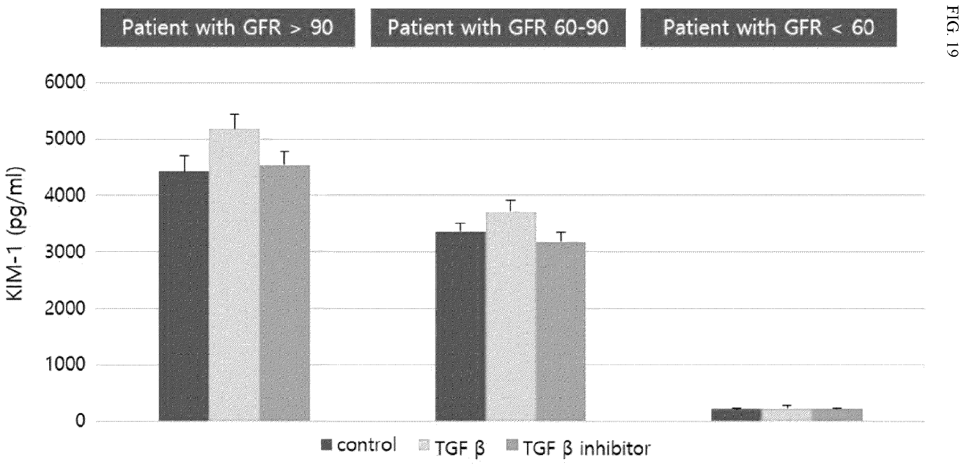

FIG. 19 are graphs illustrating the expression levels of KIM-1, which is a renal fibrosis evaluation index, in renal fibrosis-on-chips manufactured using cells derived from a patient with renal fibrosis having a GFR of more than 90, cells from a patient with renal fibrosis having a GFR of 60 to 90, and cells from a patient with renal fibrosis having a GFR of less than 60 according to an embodiment of the present invention before TGF-β stimulation (control), after TGF-β stimulation (TGF-β), and after TGF-β inhibitor administration (TGF-β inhibitor); and

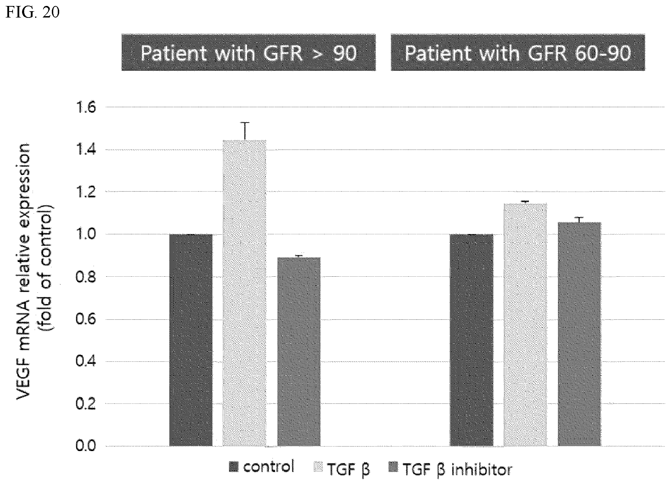

FIG. 20 are graphs illustrating the expression levels of VEGF, which is a renal fibrosis evaluation index, in renal fibrosis-on-chips manufactured using cells derived from a patient with renal fibrosis having a GFR of more than 90 and cells from a patient with renal fibrosis having a GFR of 60 to 90 according to an embodiment of the present invention before TGF-β stimulation (control), after TGF-β stimulation (TGF-β), and after TGF-β inhibitor administration (TGF-β inhibitor).

### [Description of Embodiments]

Hereinafter, the present invention will be described in detail.

In an aspect of the present invention, "expression of gene" is the broadest concept including transcription, translation, post-translational modification and the like.

In an aspect of the present invention, "relative expression level" may be the degree to which expression is promoted when the expression level of a specific gene or protein, specifically one or more selected from the group consisting of CCK8, α-SMA, KIM-1, and VEGF according to an aspect of the present invention in a kidney-on-a-chip, specifically in cells or culture solution of the kidney-on-a-chip before treatment with a test substance is compared to the expression level in the cells or culture solution after treatment with the test substance. Alternatively, "relative expression level" may be the degree to which expression is promoted when the expression level of a specific gene or protein, specifically one or more selected from the group consisting of CCK8, α-SMA, KIM-1, and VEGF according to an aspect of the present invention in a kidney-on-a-chip, specifically in cells or culture solution of the kidney-on-a-chip that is treated with a test substance is compared to the expression level in a kidney-on-a-chip that is not treated with the test substance, specifically in the cells or culture solution of the kidney-on-a-chip. The relative expression level may include, for example, the relative expression level of mRNA or the relative expression level of protein.

In an aspect, the present invention provides a kidney-on-a-chip comprising a renal tubule culture part where renal tubular epithelial cells are cultured; and an interstitium culture part where renal fibroblasts and vascular endothelial cells are cultured, in which the renal tubule culture part and the interstitium culture part are horizontally arranged and the renal tubular epithelial cells, renal fibroblasts, and vascular endothelial cells are co-cultured.

Existing kidney-on-chips have a vertical structure, so a separate culture solution feeding channel is required when renal tubular epithelial cells and renal fibroblasts are co-cultured, and have a limitation that there should be no connection between the channel and the upper part. However, the kidney-on-a-chip according to an aspect of the present invention has an excellent effect of facilitating drug administration to each culture part without a separate culture solution feeding channel since the kidney-on-a-chip has a simple structure and the renal tubule culture part and the interstitium culture part may be horizontally arranged.

The interstitium culture part according to an aspect of the present invention may contain fibrin gel, specifically, the interstitium culture part may include a renal fibroblast region containing the renal fibroblasts; and a vascular endothelial cell region containing the vascular endothelial cells, more specifically, the renal fibroblast region or the vascular endothelial cell region may further contain fibrin gel. Existing kidney-on-chips have problems of structural deformation due to contraction of collagen gel when collagen gel is used and problems of culture solution leakage and cell detachment from the membrane since a separate membrane is contained, but the kidney-on-a-chip according to an aspect of the present invention does not undergo physical deformation since fibrin gel is used instead of collagen gel, does not have problems of culture solution leakage since a membrane is not contained, and has an excellent effect of simultaneously culturing renal tubular epithelial cells, renal fibroblasts, and vascular endothelial cells since the cell adhesion ability is maintained on fibrin gel as well.

One or more cells selected from the group consisting of the renal tubular epithelial cells, renal fibroblasts, and vascular endothelial cells according to an aspect of the present invention may be human-derived cells. In other words, in the kidney-on-a-chip according to an aspect of the present invention, two or more cells derived from renal tubular epithelial cells, renal fibroblasts, and vascular endothelial cells derived from the human body may be co-cultured. Specifically, the renal tubular epithelial cells and renal fibroblasts according to an aspect of the present invention may be human-derived cells. The human-derived cells according to an aspect of the present invention may be cells isolated from human-derived kidney tissue by methods known in the art, and the isolation method is not limited as long as human-derived cells can be co-cultured in the kidney-on-a-chip while maintaining the original characteristics and functions.

According to an embodiment of the present invention, it has been confirmed that only cytokeratin 8 (CCK8), an epithelial cell marker, is expressed but α-smooth muscle actin (α-SMA) is not expressed in the renal tubule culture part of a kidney-on-a-chip manufactured using renal tubular epithelial cells and renal fibroblasts isolated from normal human kidney tissue as well as only α-SMA, a fibroblast marker, and CD31, a vascular endothelial cell marker, are expressed but CCK8 is not expressed in the interstitium culture part. Hence, it has been found that the kidney-on-a-chip according to an aspect of the present invention has an excellent effect of saving time and cost since human-derived cells are contained and the different types of cells can be co-cultured in the kidney-on-a-chip (Example 2).

The renal tubular epithelial cells and renal fibroblasts according to an aspect of the present invention may be cells derived from a patient with kidney disease, specifically may be cells derived from a patient with advanced renal fibrosis, and more specifically may be cells derived from a patient with renal failure, but are not limited thereto. In the kidney-on-a-chip according to an aspect of the present invention, cells derived from renal tubular epithelial cells or renal fibroblasts derived from a patient with kidney disease can be co-cultured, and the kidney-on-a-chip in which renal tubular epithelial cells or renal fibroblasts derived from a patient with kidney disease are contained or co-cultured may be a renal fibrosis-on-a-chip. The renal tubular epithelial cells and renal fibroblasts according to an aspect of the present invention may be cells derived from a patient with kidney disease, and the cells derived from a patient with kidney disease may be cells isolated from kidney tissue derived from a patient with kidney disease by methods known in the art, and the isolation method is not limited as long as cells derived from a patient with kidney disease can be co-cultured in the kidney-on-a-chip while maintaining the original characteristics and functions.

According to an embodiment of the present invention, it has been confirmed that only cytokeratin 8 (CCK8), an epithelial cell marker, is expressed but α-smooth muscle actin (α-SMA) is not expressed in the renal tubule culture part of a renal fibrosis-on-a-chip manufactured using renal fibroblasts isolated from kidney tissue derived from a patient with renal failure as well as fluorescence expression of vascular endothelial cells is confirmed but α-SMA is not expressed in the vascular endothelial cell region of the interstitium culture part and the formation of capillary structures is confirmed in this region. Hence, it has been found that the kidney-on-a-chip according to an aspect of the present invention can be used as a model to screen for renal fibrosis therapeutic agents and has an excellent effect of saving time and cost for the development of therapeutic agents since cells derived from a patient with kidney disease are contained and the different types of cells can be co-cultured in the kidney-on-a-chip (Example 3).

One or more cells selected from the group consisting of the renal tubular epithelial cells, renal fibroblasts, and vascular endothelial cells according to an aspect of the present invention may be cells undergoing fluorescence staining. In other words, in the kidney-on-a-chip according to an aspect of the present invention, one or more cells derived from renal tubular epithelial cells, renal fibroblasts, and vascular endothelial cells can undergo fluorescence staining, specifically one or more cells selected from the group consisting of the renal tubular epithelial cells, renal fibroblasts, and vascular endothelial cells may be cells undergoing immunofluorescence staining, and the method is not limited as long as it is a cell staining method that can confirm whether the different cells co-cultured are live or dead. In other words, in the kidney-on-a-chip according to an aspect of the present invention, one or more cells derived from renal tubular epithelial cells, renal fibroblasts, and vascular endothelial cells may undergo fluorescence staining. Alternatively, one or more cells selected from the group consisting of the renal tubular epithelial cells, renal fibroblasts, and vascular endothelial cells according to an aspect of the present invention may be cells that express fluorescence. According to an embodiment of the present invention, it has been found that in the kidney-on-a-chip according to an aspect of the present invention, the renal tubule culture part and the interstitium culture part are horizontally arranged and the structure is simplified and well divided, and thus the efficiency of substance exchange among the three types of cells and fluorescence staining is increased without including a separate culture solution feeding channel (Examples 1-1 and 2-2).

The kidney-on-a-chip according to an aspect of the present invention may include two or more interstitium culture parts, and the renal tubule culture part may be located between the two or more interstitium culture parts. For example, when the two interstitium culture parts are arranged horizontally, the renal tubule culture part may be disposed between these (FIGS. 6A and 12A).

In another aspect, the present invention provides a method for manufacturing the kidney-on-a-chip described above, the method comprising culturing renal tubular epithelial cells in the renal tubule culture part and culturing renal fibroblasts and vascular endothelial cells in the interstitium culture part. Descriptions of the kidney-on-a-chip, renal tubule culture part, renal tubular epithelial cells, interstitium culture part, renal fibroblasts, vascular endothelial cells, and the like are as described above.

The manufacturing method according to an aspect of the present invention may further include a step of isolating renal tubular epithelial cells and renal fibroblasts from human kidney tissue before the cell culturing step, and the isolation may be performed by methods known in the art.

In still another aspect, the present invention provides a co-culture device comprising a renal tubule culture part where renal tubular epithelial cells are cultured; and an interstitium culture part where renal fibroblasts and vascular endothelial cells are cultured, in which the renal tubule culture part and the interstitium culture part are horizontally arranged and the renal tubular epithelial cells, renal fibroblasts, and vascular endothelial cells are co-cultured. Descriptions of the renal tubular epithelial cells, renal tubule culture part, renal fibroblasts, vascular endothelial cells, interstitium culture part, horizontal arrangement, and the like are as described above.

In still another aspect, the present invention provides a method for screening for a renal fibrosis therapeutic agent, the method comprising (a) treating the kidney-on-a-chip described above with transforming growth factor-β (TGF-β); (b) treating the kidney-on-a-chip with a test substance; and (c) measuring a renal fibrosis evaluation index in the kidney-on-a-chip treated with the test substance. The description of the kidney-on-a-chip is as described above.

The step (a) of treating TGF-β according to an aspect of the present invention may be adding TGF-β to the culture solution in the kidney-on-a-chip, and the amount or concentration of TGF-β added may vary depending on the amount of cells present in the kidney-on-a-chip, the amount of test substance, or the like, and is not limited as long as it is an amount or concentration in which a renal fibrosis therapeutic agent can be screened for without damaging or killing cells present in the kidney-on-a-chip. In the kidney-on-a-chip according to an aspect of the present invention, different types of cells, including renal tubular epithelial cells, renal fibroblasts, and vascular endothelial cells, can be co-cultured, and TGF-β further stimulates renal fibroblasts when the cells are co-cultured and treated with TGF-β compared to when each of the cells are separately cultured and treated with TGF-β, so more various cytokines (for example, fibroblast growth factor (FGF), interleukin-1β (IL-1β), TGF-β2, TGF-β3, and vascular endothelial growth factor (VEGF)) are amplified together, and the kidney-on-a-chip can mimics the *in vivo* environment more favorably.

In an embodiment of the present invention, one or more cells selected from the group consisting of the renal tubular epithelial cells, renal fibroblasts, and vascular endothelial cells in the kidney-on-a-chip may be cells derived from a patient with kidney disease, specifically may be cells derived from a patient with advanced renal fibrosis, and more specifically may be cells derived from a patient with renal failure, but are not limited thereto. In other words, in the kidney-on-a-chip according to an aspect of the present invention, one or more cells selected from the group consisting of renal tubular epithelial cells, renal fibroblasts, and vascular endothelial cells derived from a patient with kidney disease can be co-cultured, and the kidney-on-a-chip in which one or more cells selected from the group consisting of renal tubular epithelial cells, renal fibroblasts, and vascular endothelial cells derived from a patient with kidney disease are co-cultured may be a renal fibrosis-on-a-chip. One or more cells selected from the group consisting of the renal tubular epithelial cells, renal fibroblasts, and vascular endothelial cells according to an aspect of the present invention may be cells derived from a patient with kidney disease, and the cells derived from a patient with kidney disease may be cells isolated from kidney tissue derived from a patient with kidney disease by methods known in the art, and the isolation method is not limited as long as cells derived from a patient with kidney disease can screen for renal fibrosis therapeutic agents while maintaining the original characteristics and functions.

According to an embodiment of the present invention, it can be seen that a renal fibrosis-on-a-chip manufactured using renal fibroblasts isolated from kidney tissue derived from a patient with renal failure has an excellent effect of efficiently screening for renal fibrosis therapeutic agents in terms of time and cost since there are differences in renal fibrosis evaluation indices before and after treatment with a test substance for renal fibrosis treatment or between an organ-on-a-chip not treated with the test substance and an organ-on-a-chip (renal fibrosis-on-a-chip) treated with the test substance (Experimental Examples 1 to 3).

The step (c) of measuring a renal fibrosis evaluation index according to an aspect of the present invention may include comparing a renal fibrosis evaluation index before the kidney-on-a-chip is treated with a test substance to the renal fibrosis evaluation index after the kidney-on-a-chip is treated with the test substance or a renal fibrosis evaluation index when the kidney-on-a-chip is not treated with a test substance to the renal fibrosis evaluation index when the kidney-on-a-chip is treated with the test substance. The step (c) of measuring a renal fibrosis evaluation index according to an aspect of the present invention may include comparing a renal fibrosis evaluation index in a kidney-on-a-chip that is treated with a test substance to the renal fibrosis evaluation index in a kidney-on-a-chip that is not treated with the test substance or is not yet treated with the test substance.

The renal fibrosis evaluation index according to an aspect of the present invention may be one or more selected from the group consisting of relative expression levels of one or more selected from the group consisting of cytokeratin 8 (CCK8) and α-smooth muscle actin (α-SMA) in the renal tubular epithelial cells; the degree of angiogenesis in the interstitium culture part where vascular endothelial cells are cultured; relative expression level of cluster of differentiation 31 (CD31) in the vascular endothelial cells; and relative expression levels of one or more selected from the group consisting of kidney injury molecule-1 (KIM-1) and vascular endothelial growth factor (VEGF) in the culture solution, but is not limited to this as long as it is an objective evaluation index that can determine a renal fibrosis therapeutic agent as the results vary depending on whether the kidney-on-a-chip according to an aspect of the present invention is treated with a test substance. The culture solution according to an aspect of the present invention may be a cell culture solution present in the kidney-on-a-chip, specifically may be a culture solution for co-culturing renal tubular epithelial cells, renal fibroblasts, and vascular endothelial cells, and more specifically may be a vascular endothelial cell culture solution.

The step (c) of measuring a renal fibrosis evaluation index according to an aspect of the present invention may include measuring relative expression levels of one or more selected from the group consisting of CCK8 and α-SMA in the renal tubular epithelial cells. The screening method according to an aspect of the present invention may further include a step of determining the test substance as a renal fibrosis therapeutic agent when the expression level of CCK8 after treatment with the test substance is increased or the expression level of α-SMA after treatment with the test substance is decreased compared to the expression level of CCK8 or the expression level of α-SMA without treatment with the test substance or before treatment with the test substance as a result of measuring the relative expression levels in step (c).

In the renal tubule culture part containing renal tubular epithelial cells, the expression level of CCK8 decreases when renal fibrosis is induced in the kidney-on-a-chip according to an embodiment of the present invention through TGF-β stimulation, but does not change or increases when a TGF-β inhibitor, a type of renal fibrosis therapeutic agent, is administered compared to that before TGF-β stimulation, and increases compared to that at the time of TGF-β stimulation. Hence, it has been found that the tendency to decrease the expression level of CCK8 due to renal fibrosis induced through TGF-β stimulation is offset by the administration of TGF-β inhibitor and the relative expression level of CCK8 in the renal tubular epithelial cells can be utilized as an evaluation index for screening for renal fibrosis therapeutic agents (Experimental Examples 1 and 2).

In the renal tubule culture part containing renal tubular epithelial cells, the expression level of α-SMA increases when renal fibrosis is induced in the kidney-on-a-chip according to an embodiment of the present invention through TGF-β stimulation, but does not change or decreases when a TGF-β inhibitor, a type of renal fibrosis therapeutic agent, is administered compared to that before TGF-β stimulation, and decreases compared to that at the time of TGF-β stimulation. Hence, it has been found that the tendency to increase the expression level of α-SMA due to renal fibrosis induced through TGF-β stimulation is offset by the administration of TGF-β inhibitor and the relative expression level of α-SMA in the renal tubular epithelial cells can be utilized as an evaluation index for screening for renal fibrosis therapeutic agents (Experimental Examples 1 and 2).

The step (c) of measuring a renal fibrosis evaluation index according to an aspect of the present invention may include measuring the degree of angiogenesis in the interstitium culture part where vascular endothelial cells are cultured, and the degree of angiogenesis may be the sum of lengths of blood vessels having a thickness of less than 50 µm among blood vessels formed in the interstitium culture part. The screening method according to an aspect of the present invention may further include a step of determining the test substance as a renal fibrosis therapeutic agent when the degree of angiogenesis after treatment with the test substance is decreased compared to the degree of angiogenesis without treatment with the test substance or before treatment with the test substance as a result of measuring the degree of angiogenesis in step (c).

In the interstitium culture part containing vascular endothelial cells, the degree of angiogenesis increases as the total length of thick blood vessels decreases, the diameter of thick blood vessels also decreases, and the total length of thin blood vessels also increases when renal fibrosis is induced in the kidney-on-a-chip according to an embodiment of the present invention through TGF-β stimulation, but does not change or decreases when a TGF-β inhibitor, a type of renal fibrosis therapeutic agent, is administered compared to that before TGF-β stimulation, and decreases compared to that at the time of TGF-β stimulation. Hence, it has been found that the tendency to increase the degree of angiogenesis due to renal fibrosis induced through TGF-β stimulation is offset by the administration of TGF-β inhibitor and changes in the degree of angiogenesis in the interstitium culture part containing vascular endothelial cells can be utilized as an evaluation index for screening for renal fibrosis therapeutic agents (Experimental Examples 1 and 2).

The step (c) of measuring a renal fibrosis evaluation index according to an aspect of the present invention may include measuring the relative expression level of cluster of differentiation 31 (CD31) in the vascular endothelial cells. The screening method according to an aspect of the present invention may further include a step of determining the test substance as a renal fibrosis therapeutic agent when the relative expression level of CD31 after treatment with the test substance is decreased compared to the relative expression level of CD31 without treatment with the test substance or before treatment with the test substance as a result of measuring the relative expression level in step (c).

In the interstitium culture part containing vascular endothelial cells, α-SMA from renal tubular epithelial cells invades the vascular endothelial cell region and the expression level of α-SMA increases when renal fibrosis is induced in the kidney-on-a-chip according to an embodiment of the present invention through TGF-β stimulation, but does not change or decreases when a TGF-β inhibitor, a type of renal fibrosis therapeutic agent, is administered compared to that before TGF-β stimulation, and decreases compared to that at the time of TGF-β stimulation. Hence, it has been found that the tendency to increase the expression level of α-SMA due to renal fibrosis induced through TGF-β stimulation is offset by the administration of TGF-β inhibitor and the relative expression level of α-SMA in the renal tubular epithelial cells can be utilized as an evaluation index for screening for renal fibrosis therapeutic agents (Experimental Examples 1 and 2). According to an embodiment of the present invention, it has been confirmed that TGF-β induces epithelial mesenchymal transition (EMT), followed by invasion of renal tubular epithelial cells into the interstitium, and it has been confirmed that humoral factors of renal fibroblasts are involved in this mechanism (Experimental Examples 1 and 2).

In the interstitium culture part containing vascular endothelial cells, the expression level of VEGF increases when renal fibrosis is induced in the kidney-on-a-chip according to an embodiment of the present invention through TGF-β stimulation, but decreases when a TGF-β inhibitor, a type of renal fibrosis therapeutic agent, is administered. Hence, it has been found that the tendency to increase the expression level of VEGF due to renal fibrosis induced through TGF-β stimulation is offset by the administration of TGF-β inhibitor and the relative expression level of VEGF in the interstitium culture part containing vascular endothelial cells can be utilized as an evaluation index for screening for renal fibrosis therapeutic agents (Experimental Example 3).

The step (c) of measuring a renal fibrosis evaluation index according to an aspect of the present invention may include measuring relative expression levels of one or more selected from the group consisting of kidney injury molecule-1 (KIM-1) and vascular endothelial growth factor (VEGF) in the culture solution contained in the kidney-on-a-chip. The screening method according to an aspect of the present invention may further include a step of determining the test substance as a renal fibrosis therapeutic agent when expression levels of one or more selected from the group consisting of KIM-1 and VEGF after treatment with the test substance are decreased compared to the expression levels without treatment with the test substance or before treatment with the test substance as a result of measuring the relative expression levels in step (c).

In the culture solution, the expression level of KIM-1 increases when renal fibrosis is induced in the kidney-on-a-chip according to an embodiment of the present invention through TGF-β stimulation, but does not change or decreases when a TGF-β inhibitor, a type of renal fibrosis therapeutic agent, is administered compared to that before TGF-β stimulation, and decreases compared to that at the time of TGF-β stimulation. Hence, it has been found that the tendency to decrease the expression level of KIM-1 due to renal fibrosis induced through TGF-β stimulation is offset by the administration of TGF-β inhibitor and the relative expression level of KIM-1 in the culture solution can be utilized as an evaluation index for screening for renal fibrosis therapeutic agents (Experimental Example 3).

### [Examples]

Hereinafter, the configuration and effects of the present invention will be described more specifically with reference to Examples and Experimental Examples. However, the following Examples and Experimental Examples are provided only for illustrative purposes to aid understanding of the present invention, and the gist and scope of the present invention are not limited thereto.

### [Example 1] Development of kidney-on-a-chip using rodent-derived renal tubular epithelial cells and renal fibroblasts

Conventionally, a kidney-on-a-chip having a vertical multilayer structure exists for co-culturing rodent-derived renal tubular epithelial cells (NRK 52) and renal fibroblasts (NRK 49) (FIG. 1A), but has problems that a separate culture solution feeding channel is additionally required for three-dimensional fibroblast culture when hydrogel is used and the structure of the lower layer is not constantly maintained because of contraction of collagen gel when collagen gel is used. Renal fibroblasts (NRK 49) are cultured in a three dimensional manner using fibrin gel for the purpose of constantly maintaining a lower layer structure, but there is a limitation that cells cannot proliferate smoothly since the culture solution is not supplied smoothly. Furthermore, there is a structural problem that since three dimensional fibroblasts (NRK-49F) do not proliferate properly when the culture solution is supplied only from one side although the culture solution is supplied from the epithelial cell side, and the culture solution feeding channel is required to be additionally provided to supply the culture solution to the side where three dimensional fibroblasts are located for three dimensional fibroblast (NRK-49F) culture.

Accordingly, a kidney-on-a-chip to smoothly administer culture solutions required for the proliferation of renal fibroblasts and therapeutic agent candidates and to solve the structural problems as described above was manufactured in the following manner.

### [Example 1-1] Manufacture of kidney-on-a-chip using rodent-derived renal tubular epithelial cells and renal fibroblasts

The kidney-on-a-chip according to an aspect of the present invention was manufactured in the following manner using rodent-derived renal tubular epithelial cells (NRK-52E) (ATCC #CRL-1571) and renal fibroblasts (NRK-49F) (ATCC #CRL-1570) and DMEM high glucose medium (Welgene #LM001-51) supplemented with 5% bovine calf serum (Gibco #16170-078) and 1% penicillin-streptomycin (Gibco #15140-122) as the culture solution for these.

The reagents used in the preparation of fibrin gel used in the organ-on-a-chip are as follows.
- Fibrinogen from bovine plasma (Sigma, #F8630): Fibrinogen was dissolved at 10 mg/ml in 1x PBS, and the solution was mixed gently (did not form a vortex), incubated in a 37°C water bath for 30 minutes, and then filtered through a sterile filter (0.22 µm filter).
- Thrombin from bovine plasma (Sigma, #T4648 1KU): Thrombin was dissolved in 3'DW at 50 U/ml, and the solution was filtered through a sterile filter (0.22 µm filter).
- Aprotinin from bovine lung (Sigma, #A1153): Aprotinin was dissolved in 3'DW at 4 TIU/ml, and the solution was filtered through a sterile filter (0.22 µm filter).
- Each was prepared in an aliquot of 500 µl and stored at -20°C.

Blank fibrin gel and Fibrin gel+Cell were prepared as follows. In a 1.5 ml E-tube, 250 µl of fibrinogen was placed, and then 40 µl of aprotinin was added into the E-tube. At this time, the blank fibrin gel (fibrin at 2.5 mg/ml) contains 250 µl of fibrinogen at 10 mg/ml, 40 µl of aprotinin at 4 TIU/ml, and 710 µl of 1x PBS, and the fibrinogen and aprotinin solution contains 250 µl of fibrinogen at 10 mg/ml and 40 µl of aprotinin at 4 TIU/ml. Next, before cell preparation, aprotinin and thrombin were taken out of the freezer and thawed at room temperature. Afterwards, 37.5 µl of cell suspension in which ratio of the cell suspension to the fibrinogen and aprotinin solution was 3:1 was dispensed into each E-tube. At this time, the concentration of cells should be higher than the target concentration by 4/3-fold. Then, 1 µl of thrombin was loaded into each well of a 96-well plate, 12.5 µl of the fibrinogen and aprotinin solution was added into the prepared E-tube (containing 37.5 µl of cell suspension), and gentle mixing was performed. The mixture (50 µl in total) was transferred to the wells preloaded with 1 µl of thrombin, and gentle and quick mixing was performed by pipetting this up and down two times. From the mixture, 20 µl or 50 µl was taken, mixed gently, and then injected into the gel loading port of the cell culture channel (fibrin coagulation within 45 to 60 seconds), whereby a kidney-on-a-chip was manufactured using rodent-derived renal tubular epithelial cells and renal fibroblasts.

As illustrated in FIG. 1B, the kidney-on-a-chip manufactured by the method maintained the structure in which renal tubular epithelial cells and renal fibroblasts were co-cultured in a three dimensional manner, but the design of the kidney-on-a-chip was changed from the conventional vertical structure to a horizontal structure in which feeding supply of the culture solution to each compartment is separately maintained. As illustrated in FIG. 4A, the manufactured kidney-on-a-chip has a horizontal structure and is divided into a total of 5 compartments, and the compartment is divided by the blank fibrin gel located in the middle and is divided into a renal tubular epithelial cell culture compartment (Epithelial cell Suspension in FIG. 4A) and a renal fibroblast culture compartment (Fibroblast + Fibrin gel in FIG. 4A) at the top and bottom. Furthermore, each compartment was divided without constructing a separate membrane and the culture solution was easily administered by utilizing fibrin gel exhibiting low contractility in the renal fibroblast culture space, whereby co-culture conditions were completed.

### [Example 1-2] Confirmation of biomimeticity

The following experiment was performed to examine whether the kidney-on-a-chip manufactured in Example 1-1 normally mimics a living body.

### A. Confirmation of cell adhesion and proliferation abilities of cells when cultured on fibrin gel

Conventionally, problems such as culture solution leakage or cell detachment were discovered in organ-on-chips manufactured to have a structure in which a separate membrane was inserted between PDMS materials, and the kidney-on-a-chip manufactured in Example 1-1 was divided into compartments using blank fibrin gel to supplement this.

First, an experiment was performed to examine whether there were differences in adhesion and proliferation abilities between renal tubular epithelial cells (NRK-52E) cultured in regular wells and the cells cultured on fibrin gel. DMEM high glucose medium (Welgene #LM001-51) supplemented with 5% bovine calf serum (Gibco #16170-078) and 1% penicillin-streptomycin (Gibco #15140-122) was used as a culture solution for renal tubular epithelial cells (NRK-52E)(ATCC #CRL-1571), fibrin gel was prepared using fibrinogen (Sigma, #F8630), thrombin (Sigma, #T4648 1KU), and aprotinin (Sigma, #A1153), and the survival/death of cells was examined through staining using the LIVE/DEAD Viability/Cytotoxicity Kit (Invitrogen, #L3224) for mammalian cells. In the case of culture in regular wells (control group, control in FIG. 3), the prepared renal tubular epithelial cells were seeded in a 96-well plate at 2 × 10⁴/well and cultured for 3 days. In the case of culture on fibrin gel (Fibrin gel in FIG. 3), a blank fibrin gel solution containing 250 µl of fibrinogen at 10 mg/ml, 40 µl of aprotinin at 4 TIU/ml, and 710 µl of 1x PBS was prepared, and 50 µl of the blank fibrin gel solution and 1 µl of thrombin were mixed, placed in the wells of a 96-well plate, and subjected to gelling for about 3 to 5 minutes. Next, the cells were seeded at 2 × 10⁴/well and cultured for 3 days. Thereafter, to the control group and cells cultured in fibrin gel, 100 µl of the following were placed in each well and cultured at room temperature for 30 minutes: Hoechst at 1:500 for stain cell nuclei staining, Calcein at 1:2000 for live cell staining, and EthD-1 at 1:500 for dead cell staining. Then, washing was performed with 1x PBS, photographing was performed using a confocal microscope, and the results are as illustrated in FIG. 3.

As illustrated in FIG. 3, it has been confirmed that there is no difference in adhesion and proliferation abilities between cells cultured in regular wells and cells cultured on fibrin gel.

### B. Confirmation of renal tubular epithelial cell adhesion and proliferation abilities on fibrin gel of kidney-on-a-chip

An experiment was performed to examine whether renal tubular epithelial cells (NRK-52E) adhered to fibrin gel in a monolayer and were cultured. The renal tubular epithelial cells, fibrin gel, and renal fibroblasts used at this time were the same as those in Example 1-1.

A mixture of 50 µl of blank fibrin gel at 2.5 mg/ml in which 250 µl of fibrinogen at 10 mg/ml, 40 µl of aprotinin at 4 TIU/ml, and 710 µl of 1x PBS were mixed and 1 µl of thrombin was seeded in the blank fibrin gel region (yellow region in FIG. 4C, Gel A) of the kidney-on-a-chip manufactured in Example 1-1 as i and ii in FIG. 4D, 12.5 µl of fibrin gel solution at 10 mg/ml in which 250 µl of fibrinogen at 10 mg/ml and 40 µl of aprotinin at 4 TIU/ml were mixed was mixed with 37.5 µl of renal fibroblasts at 200 × 10⁴/ml, 50 µl of the mixture and 1 µl of thrombin were mixed, and this was seeded in the renal fibroblast region of the kidney-on-a-chip (blue region in FIG. 4C, Gel B) as iii and iv in FIG. 4D, and gelling was performed for about 3 to 5 minutes. Then, 10 µl of renal tubular epithelial cell suspension at 300 × 10⁴/ml was seeded in the upper region of the blank fibrin gel region (hereinafter referred to as renal tubular epithelial cell region) of the kidney-on-a-chip as v in FIG 4D, tilted 90° in the desired direction (upward in this experiment), and cultured at 37°C for 30 minutes. After culture, each region of the kidney-on-a-chip was filled with 150 to 200 µl of the same medium as that in the experiment to examine cell adhesion and proliferation abilities. The renal tubular epithelial cells and renal fibroblasts and the like were seeded and cultured for 7 days, then live and dead cells were stained in the same manner as in A. of Example 1-2 and photographed using a confocal microscope, and the results are as illustrated in FIG. 4E.

As illustrated in FIG. 4E, it has been confirmed that renal tubular epithelial cells adhere to the fibrin gel in a monolayer to achieve confluency, renal fibroblasts also proliferate in an independent space of the fibrin gel, and physical deformation due to additional contraction was not observed unlike organ-on-chips using collagen gel.

### C. Confirmation of co-culture of renal tubular epithelial cells and renal fibroblasts in kidney-on-a-chip

In order to examine whether co-culture of renal tubular epithelial cells and renal fibroblasts is performed smoothly in the kidney-on-a-chip manufactured in Example 1-1, immunofluorescence staining (IF staining) was performed on E-cadherin, an epithelial cell marker. The renal tubular epithelial cells, fibrin gel, and renal fibroblasts used at this time were the same as those in Example 1-1, and their seeding and culturing methods are the same as those in B. of Example 1-2.

Specifically, cells in each of the renal fibroblast region and renal tubular epithelial cell region of the kidney-on-a-chip manufactured in Example 1-1 were fixed with 4% paraformaldehyde fixative (Biosesang, #P2031) at room temperature for 15 minutes, treated with 0.5% Triton-X (Sigma, #T9284), and cultured at room temperature for 10 minutes, then 1% bovine serum albumin (BSA) (Sigma, #A2153) was added, and culture was performed at 37°C for 30 minutes. After culture, the cells were washed with 1x PBS (Welgene, #LB004-02), treated with the primary antibody against E-cadherin (BD, #610182) diluted with 1x PBS, then reacted at room temperature for 3 days, and washed again with 1x PBS. After washing, the cells were treated with the secondary antibody against E-cadherin (Anti-Mouse IgG H&L) (Alexa Fluor 488) (Abcam, #ab150105) diluted with 1% BSA at 1:100, reacted at room temperature overnight, and then washed again with 1x PBS. The cells were then treated with the dye Hoechst diluted with 1x PBS at 1:250 and stained at room temperature for 3 hours, washed with 1x PBS, and then photographed using a confocal microscope, and the results are illustrated in FIG. 5.

As illustrated in FIG. 5, it has been confirmed that E-cadherin is normally and clearly expressed in the renal tubular epithelial cell region of the kidney-on-a-chip manufactured in Example 1-1, and E-cadherin is not expressed in the renal fibroblast region. Therefore, it has been found that renal tubular epithelial cells and renal fibroblasts can be co-cultured using the kidney-on-a-chip manufactured in Example 1-1.

Through this, it has been confirmed that in the kidney-on-a-chip manufactured in Example 1-1, renal fibroblasts can be cultured in a three dimensional manner by using fibrin gel to minimize physical deformation and proliferation is maintained without physical deformation at this time, and it has been confirmed that it is easy to administer drugs to each compartment without having a separate culture solution feeding channel by securing five compartments in a horizontal structure. In addition, it has been confirmed that there is an advantage of preventing leakage of culture solution even without using a membrane, engraftment of renal tubular epithelial cells is easy, co-culture of renal tubular epithelial cells and renal fibroblasts is possible to enable seeding two types of cells at the same time, and thus there is an excellent effect of shortening the organ-on-a-chip manufacturing period.

### [Example 2] Development of kidney-on-a-chip using human-derived renal tubular epithelial cells and renal fibroblasts

Existing organ-on-chips using pumps have limitations in introducing various types of cells and do not allow high throughput, so there are disadvantages in applying a variety of experimental groups in a short period of time. In addition, there are gaps between the respective compartments in the horizontal structure of the organ-on-chips, so there is a problem that a lot of time is required for cell-cell substance exchange and cell staining (immunofluorescence staining and the like). Moreover, in addition to renal tubular epithelial cells and renal fibroblasts, which are the main cells that cause renal fibrosis, vascular endothelial cells are known to be a major factor participating in the progression of fibrosis, so the introduction of vascular endothelial cells is necessary to reflect this.

Accordingly, the organ-on-a-chip manufactured in Example 1-1 was improved by reflecting the problems, and a kidney-on-a-chip using human-derived cells was manufactured as follows.

### [Example 2-1] Isolation of renal tubular epithelial cells and renal fibroblasts from human kidney tissue

In order to isolate renal tubular epithelial cells and renal fibroblasts from human kidney tissue, a cohort was established to obtain human-derived kidney tissue from patients undergone nephrectomy, and IRB approval was obtained (B-1408-264-003). Afterwards, some kidney tissues were obtained from four patients in Table 1 below who agreed to donate human derivatives among patients undergoing radical nephrectomy, cells were treated using tissue dissociation equipment in the following manner, and renal epithelial cells and renal fibroblasts were isolated from normal kidney tissue and identified through FACS analysis and immunofluorescence staining (IF staining).

**[Table 1]**

| Patient # | Surgery date | Kidney function of patient (eGFR) | Type of cell | Patient List | Type of cell | FACS (+)% | FACS (-)% |
|---|---|---|---|---|---|---|---|
| 1 | March 29, 2018 | Chronic renal failure (61.2) | Normal renal tubular epithelial cell | NX001-N | Epi(+) | 98.57 | 92.92 |
| | March 29, 2018 | | Normal renal fibroblast | NX001-N | Fibro(+) | 92.25 | 67.02 |
| 2 | April 03, 2018 | Normal renal function (92.4) | Normal renal tubular epithelial cell | NX003-N | Epi(+) | 97.35 | 78.31 |
| | April 03, 2018 | | Normal renal fibroblast | NX003-N | Fibro(+) | 78.13 | 58.78 |
| 3 | April 04, 2018 | Chronic renal failure (57.3) | Normal renal tubular epithelial cell | NX004-N | Epi(+) | 68.57 | 64.87 |
| | April 04, 2018 | | Normal renal fibroblast | NX004-N | Fibro(+) | 53.36 | 43.37 |
| 4 | June 12, 2018 | Normal renal function (101.7) | Normal renal tubular epithelial cell | NX010-N | Epi(+) | 97.11 | 90.21 |
| | June 12, 2018 | | Normal renal fibroblast | NX010-N | Fibro(+) | 87.87 | 44.12 |

Specifically, the kidney tissues obtained from the patients in Table 1 above were stored at 4°C, then unnecessary parts were removed from each tissue, the kidney tissues were divided into 0.5 g or less and placed in an E-tube, and the E-tube was inserted into ice. Afterwards, 2.5 ml of Normal Tissue Enzyme Mix was added into a C-tube, and each tissue was washed with PBS, then placed in the C-tube, and cut with scissors to a size of about 3 to 4 mm. The C-tube was inserted into a tissue grinder (gentleMACSTM Octo Dissociator, Miltenyi Biotec, #130-095-937), and the tissue was separated into single cells using a program appropriate for the tissue (Normal-37C_Multi_B). Next, the separated cells were filtered through a strainer, washed, and divided into cells according to the type of cell culture medium, each cell was cultured to some extent, then renal tubular epithelial cells were labeled with CD326 (EpCAM) MicroBeads human (Miltenyi Biotec, #130-061-101), renal fibroblasts were labeled with Anti-Fibroblast MicroBeads human (Miltenyi Biotec, #130-050-601), and then each labeled cell was separated using a cell separator (MidiMACS^{™} separator, Miltenyi Biotec, #130-042-302) and LS Separation Columns (Miltenyi Biotec, #130-042-401). In order to identify the separated cells, renal tubular epithelial cells were treated with CD326(EpCAM)-PE antibody, renal fibroblasts were treated with Anti-Fibroblast-PE antibody, and then FACS analysis was performed, and as a result, it has been confirmed that renal tubular epithelial cells and renal fibroblasts are each separated (FACS analysis graph in FIG. 7). In order to perform immunofluorescence staining, the isolated renal fibroblasts and renal tubular epithelial cells and two types of vascular endothelial cells (LONZA #C-2519A, HUVEC-Umbil Vein, Pooled Cells for HUVEC; ANGIO-PROTEOMIE #cAP-0001GFP, GFP Expressing Human Umbilical Vein Endotheial Cell for GFP-HUVEC) were each fixed with 4% paraformaldehyde fixative (Biosesang, #P2031) at room temperature for 20 minutes, washed with 1x PBS, then treated with 0.5% Triton-X (Sigma, #T9284), and cultured at room temperature for 20 minutes. Next, the cells were treated with primary antibodies (α-SMA antibody for renal fibroblasts, E-cadherin antibody and cytokeratin 8 (CCK8) antibody for renal tubular epithelial cells, and CD-31 antibody for vascular endothelial cells) diluted with 1% BSA, cultured overnight at 4°C, and washed again with 1x PBS. After washing, the cells were treated with secondary antibodies against each marker diluted with 1% BSA at 1:100, cultured at 4°C for 2 hours, and then washed again with 1x PBS. Afterwards, the cells were treated with Hoechst, an antibody against the three types of cells, diluted with 1x PBS at 1 :500, reacted at room temperature for 30 minutes, washed with 1x PBS, and then photographed using a confocal microscope, and the results are illustrated in FIGS. 8A to 8D. As illustrated in FIGS. 8A and 8B, it has been confirmed that only E-cadherin and cytokeratin 8, epithelial cell markers, are expressed in renal tubular epithelial cells separated by the method (FIG. 8A) and only α-smooth muscle actin (α-SMA) is expressed in renal fibroblasts. As illustrated in FIGS. 8C and 8D, it has been confirmed that cluster of differentiation 31 (CD31) is expressed in vascular endothelial cells (HUVEC, LONZA #C-2519A, HUVEC-Umbil Vein, Pooled Cells; GFP-HUVEC, ANGIO-PROTEOMIE #cAP-0001GFP, GFP Expressing Human Umbilical Vein Endotheial Cell) to be used below.

### [Example 2-2] Manufacture of kidney-on-a-chip using human-derived renal tubular epithelial cells and renal fibroblasts and confirmation of biomimeticity

### A. Manufacture of kidney-on-a-chip using human-derived renal tubular epithelial cells and renal fibroblasts

A kidney-on-a-chip, as illustrated in the schematic diagram on the right of FIG. 6A, was manufactured using human-derived renal tubular epithelial cells and renal fibroblasts obtained in Example 2-1 and vascular endothelial cells (HUVEC)(LONZA #C-2519A, HUVEC-Umbil Vein, Pooled Cells).

At this time, primary human renal fibroblasts at 400 × 10⁴/ml obtained in Example 2-1, vascular endothelial cells (HUVEC) (LONZA #C-2519A, HUVEC-Umbil Vein, Pooled Cells) at 2000 × 10⁴/ml, and fibrin gel at 10 mg/ml were seeded in the renal fibroblast and vascular endothelial cell region (yellow region in FIG. 6B, Gel A) of the kidney-on-a-chip manufactured above as i and ii in FIG. 6C and cultured at room temperature for 2 to 3 minutes. Next, 5 to 10 µl of the human-derived renal tubular fibroblast suspension at 300 × 10⁴/ml obtained in Example 2-1 was seeded in the renal tubular epithelial cell region (blue region in FIG. 6B, Gel B) of the kidney-on-a-chip as iii and iv in FIG. 6C, tilted 90° in the desired direction (upward in this experiment), and cultured at 37°C for 30 minutes. After culture, 150 to 200 µl of EGM^{™}-2 medium (LONZA, #CC-3162) was filled in each region of the kidney-on-a-chip. The renal tubular epithelial cells, vascular endothelial cells, renal fibroblasts and the like were seeded, cultured for 3 days, then treated with TGF-β at 5 ng/ml, and reacted for 24 hours.

### B. Confirmation of biomimeticity of kidney-on-a-chip using human-derived renal tubular epithelial cells and renal fibroblasts

The following immunofluorescence staining was performed to examine whether the kidney-on-a-chip manufactured above normally mimics a living body. Cells in each region of the kidney-on-a-chip were fixed with 4% paraformaldehyde fixative (Biosesang, #P2031) at room temperature for 20 minutes, washed with 1x PBS, then treated with 0.2% Triton-X (Sigma, #T9284), and cultured at room temperature for 20 minutes. The cells were then treated with 3% BSA, reacted at room temperature for 40 minutes, and then washed with 1x PBS. Next, the cells were treated with primary antibodies (α-SMA antibody (diluted at 1:100) for renal fibroblasts, cytokeratin 8 (CCK8) antibody (diluted at 1:200) for renal tubular epithelial cells, and CD-31 antibody (diluted at 1:50) for vascular endothelial cells) diluted with 1% BSA, reacted at room temperature for 3 days, and washed again with 1x PBS. After washing, the cells were treated with secondary antibodies against each marker diluted with 1% BSA at 1:100, cultured at room temperature overnight, and then washed again with 1x PBS. The cells were then treated with the antibody Hoechst diluted with 1x PBS at 1:250, reacted at room temperature for 3 hours, washed with 1x PBS, and then photographed using a confocal microscope, and the results are illustrated in FIG. 11.

As illustrated in FIG. 11, the kidney-on-a-chip manufactured by the method excluded the existing structure using a pump, and high throughput was possible. Vascular endothelial cells were introduced to more closely mimic human kidney tissue, and as a result, it has been confirmed that only α-smooth muscle actin (α-SMA) and CD31 are expressed in the renal fibroblast and vascular endothelial cell region of the kidney-on-a-chip manufactured above and only cytokeratin 8 (CCK8), an epithelial cell marker, is expressed but α-smooth muscle actin (α-SMA) is not expressed in the renal tubular epithelial cell region. As a result, it has been confirmed that human-derived renal tubular epithelial cells, renal fibroblasts, and vascular endothelial cells can be smoothly co-cultured in the kidney-on-a-chip manufactured above. In order to increase the efficiency of substance exchange among the three types of cells and immunofluorescence staining, the kidney-on-a-chip manufactured above was simplified into two compartments of a renal tubular epithelial cell compartment (renal tubule culture part) and an interstitium compartment (interstitium culture part).

Through this, it has been found that the kidney-on-a-chip manufactured in Example 2-2 has advantages that renal fibroblasts and vascular endothelial cells can be cultured simultaneously in a three dimensional manner in the interstitium culture part as the structure is simplified into a renal tubule culture part and an interstitium culture part, thus substance exchange between vascular endothelial cells and renal fibroblasts is further facilitated, and staining efficiency also is increased. In the interstitium culture part, vascular endothelial cells expressing fluorescence can be cultured, making it possible to minimize the time and errors required to screen for therapeutic agents and to secure more objective evaluation indices. In other words, it has been confirmed that the kidney-on-a-chip manufactured in Example 2-2 has an excellent effect of mimicking normal renal epithelium-vascular-fibrous tissue since microvascular structures can be formed in the kidney-on-a-chip and the structure of the kidney-on-a-chip is simplified and well divided into a renal tubule culture part and an interstitium culture part.

### [Example 3] Development of renal fibrosis-on-a-chip using renal fibroblasts derived from renal fibrosis tissue

### A. Manufacture of kidney-on-a-chip using renal fibroblasts derived from renal fibrosis tissue

It was confirmed that the kidney-on-a-chip manufactured in Example 2-2 was able to mimic a normal kidney, and thus cells were isolated from renal fibrosis tissue and an organ-on-a-chip in which the cells were cultured was manufactured in the following manner in order to further develop an organ-on-a-chip that mimicked renal fibrosis.

First, a kidney tissue sample was obtained from a patient with renal failure (Seoul National University Bundang Hospital, patient with renal cell carcinoma or renal cancer who underwent nephrectomy), and renal fibroblasts were isolated in the same manner as in Example 2-1, a renal fibrosis-on-a-chip was manufactured in the following manner, and the schematic diagram of the manufactured kidney-on-a-chip is as illustrated in FIG. 12A. At this time, in a case where human tissue-derived cells are also used for renal tubular epithelial cells, when vascular endothelial cells that fluoresce (GFP-HUVEC)(ANGIO-PROTEOMIE #cAP-0001GFP, GFP Expressing Human Umbilical Vein Endotheial Cell) are used as the vascular endothelial cells to examine biomimeticity, the cells fluoresce at the same wavelength as the marker used to identify isolated epithelial cells, making difficult to identify individual cells. Thus, unlike Example 2-2, purchased human renal tubular epithelial cells (Human Kidney-2 cells, HK-2 cells) (Korea Cell Line Bank, #22190) were used instead of the human-derived renal tubular epithelial cells.

Specifically, vascular endothelial cells (GFP-HUVEC) at 2000 × 10⁴/ml and fibrin gel at 10 mg/ml were seeded in the vascular endothelial cell region (yellow region in FIG. 12B, Gel A) of the interstitium culture part of the kidney-on-a-chip manufactured above as 1 in FIG. 12C and cultured at room temperature for 2 to 3 minutes. Next, primary human renal fibroblasts derived from a patient with renal failure at 400 × 10⁴/ml and fibrin gel at 10 mg/ml were seeded in the renal fibroblast region (red region in FIG. 12B, Gel C) of the interstitium culture part of the kidney-on-a-chip manufactured above as 2 in FIG. 12C and cultured at room temperature for 2 to 3 minutes. Afterwards, 5 to 10 µl of the human renal tubular epithelial cell (Human Kidney-2 cell, HK-2 cell) suspension at 300 × 10⁴/ml was seeded in the renal tubule culture part (blue region in FIG. 12B, Gel B) of the kidney-on-a-chip as 3 in FIG. 12C, tilted 90° in the desired direction (upward in this experiment), and cultured at 37°C for 30 minutes. After culture, each region of the kidney-on-a-chip was filled with 150 µl of the same medium as that in A. of Example 2-2. The renal tubular epithelial cells, vascular endothelial cells, renal fibroblasts and the like were seeded, cultured for 3 days, then treated with TGF-β at 5 ng/ml and TGF-β at 5 ng/ml and a TGF-β inhibitor at 10 µM, respectively, and reacted for 24 hours.

### B. Confirmation of biomimeticity of kidney-on-a-chip using renal fibroblasts derived from renal fibrosis tissue

The following immunofluorescence staining was performed to examine whether the kidney-on-a-chip manufactured above normally mimics a living body. Cells in each of the renal tubule culture part and interstitium culture part of the kidney-on-a-chip were fixed with 4% paraformaldehyde fixative (Biosesang, #P2031) at room temperature for 20 minutes, washed with 1x PBS, then treated with 0.2% Triton-X (Sigma, #T9284), and cultured at room temperature for 20 minutes. The cells were then treated with 3% BSA, reacted at room temperature for 40 minutes, and then washed with 1x PBS. Next, the cells were treated with primary antibodies (α-SMA antibody (diluted at 1:100) for renal fibroblasts and cytokeratin 8 (CCK8) antibody (diluted at 1:200) for renal tubular epithelial cells) diluted with 1% BSA, reacted at room temperature for 2 days, and washed again with 1x PBS. After washing, the cells were treated with secondary antibodies against each marker diluted with 1% BSA at 1: 100 and the antibody Hoechst diluted with 1% BSA at 1:250, reacted at room temperature for 3 hours, washed again with 1x PBS, and then photographed using a confocal microscope, and the results are illustrated in FIG. 12D.

As illustrated in FIG. 12D, it has been confirmed that only CCK8 is expressed in the renal tubule culture part (renal tubular epithelial cell region), and that fluorescence of the cells is expressed but α-SMA is hardly expressed in the vascular endothelial cell region of the interstitium culture part, confirming that capillary structures are formed in this region. It has been found that the kidney-on-a-chip according to an embodiment of the present invention has excellent effects that vascular endothelial cells can be co-cultured together with renal tubular cells and fibroblasts derived from kidney tissue with advanced renal fibrosis in addition to normal kidney tissue and the kidney-on-a-chip can mimic kidney tissue.

### [Experimental Example 1] Deduction of evaluation indices for screening for renal fibrosis therapeutic agents through comparative analysis of normal kidney-on-a-chip and renal fibrosis-on-a-chip

Through comparative analysis of an organ-on-a-chip mimicking normal kidney tissue and an organ-on-a-chip mimicking renal fibrosis, evaluation indices for screening for renal fibrosis therapeutic agents were deduced in the following manner. At this time, human-derived renal fibroblasts, vascular endothelial cells (GFP-HUVEC), and renal tubular epithelial cells were seeded in the kidney-on-a-chip (FIG. 12A) manufactured in Example 3 in the same manner as in A. of Example 3, and cultured to manufacture a kidney-on-a-chip. However, two types of renal fibroblasts of renal fibroblasts derived from normal kidney tissue (hereinafter referred to as normal kidney-on-a-chip) and renal fibroblasts derived from renal fibrosis kidney tissue (hereinafter referred to as renal fibrosis-on-a-chip) were used as the renal fibroblasts to manufacture two types of kidney-on-chips.

The two types of kidney-on-chips were divided into the control group (control in FIGS. 13A to 13D) in which each of the two types of kidney-on-chips was not treated with TGF-β and the TGF-β group (TGF β in FIGS. 13A to 13D) in which each of the two types of kidney-on-chips was treated with TGF-β unlike Example 3, and immunofluorescence staining was performed in the same manner as in B. of Example 3. At this time, the renal tubular epithelial cells in the renal tubule culture part of each kidney-on-a-chip were treated with CCK8 antibody and α-SMA antibody, respectively, their fluorescence expression was photographed using a confocal microscope (MICROSCOPE CONFOCAL LASER SCANING ZEISS LSM 710), the mean fluorescence intensity of each image was measured using the analysis program of the confocal microscope, then the result values of CCK8 and α-SMA measured for each group were compared to the values for the control group (control) to comparatively analyze the difference in expression levels, and the results are as illustrated in FIGS. 13A to 13D.

As illustrated in FIGS. 13A and 13B, in the normal kidney-on-a-chip, at the time of TGF-β stimulation, the expression level of CCK8 is decreased but the expression level of α-SMA is increased in the renal tubular epithelial cell region of the renal tubule culture part, and the number of thin blood vessels is increased in the vascular endothelial cell region of the interstitium culture part.

As illustrated in FIGS. 13C and 13D, in the renal fibrosis-on-a-chip as well, at the time of TGF-β stimulation, the expression level of CCK8 is decreased but the expression level of α-SMA is increased in the renal tubular epithelial cell region of the renal tubule culture part, and the number of thin blood vessels is increased in the vascular endothelial cell region of the interstitium culture part.

The renal tubular epithelial cells (HK-2 cells) used above undergo epithelial-mesenchymal transition (EMT) when treated with TGF-β, fibrosis progresses in the order of mesenchymal cells, myofibroblasts, and fibroblasts, and the shape of cells changes into the form of fibrosis cells. According to the results illustrated in FIGS. 13C and 13D, the expression levels of CCK and α-SMA change when TGF-β stimulates the renal fibrosis-on-a-chip, and this makes it possible to confirm the degree of EMT of renal tubular epithelial cells in the chip.

Through this, it has been confirmed that in the kidney-on-a-chip according to an embodiment of the present invention, it is possible to measure the relative expression levels of CCK8 and α-SMA in renal tubular epithelial cells present in the renal tubule culture part when TGF-β related to renal fibrosis stimulates and to measure the degree of angiogenesis through measurement of the thickness of blood vessels, and this can be utilized as an evaluation index to screen for renal fibrosis therapeutic agents.

### [Experimental Example 2] Screening for renal fibrosis therapeutic agents using kidney-on-a-chip through comparison with pathological tissue from patient with renal fibrosis

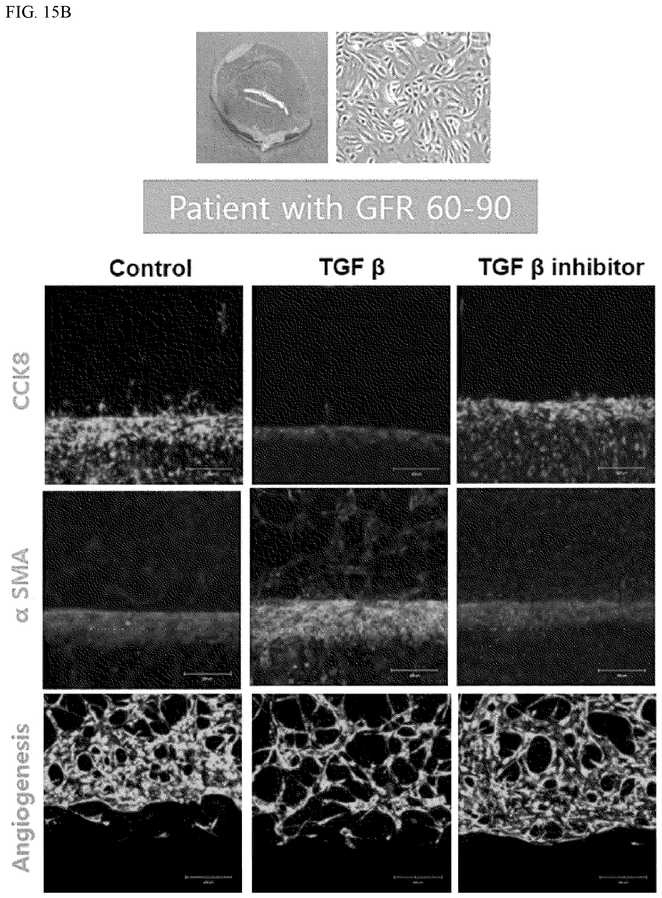

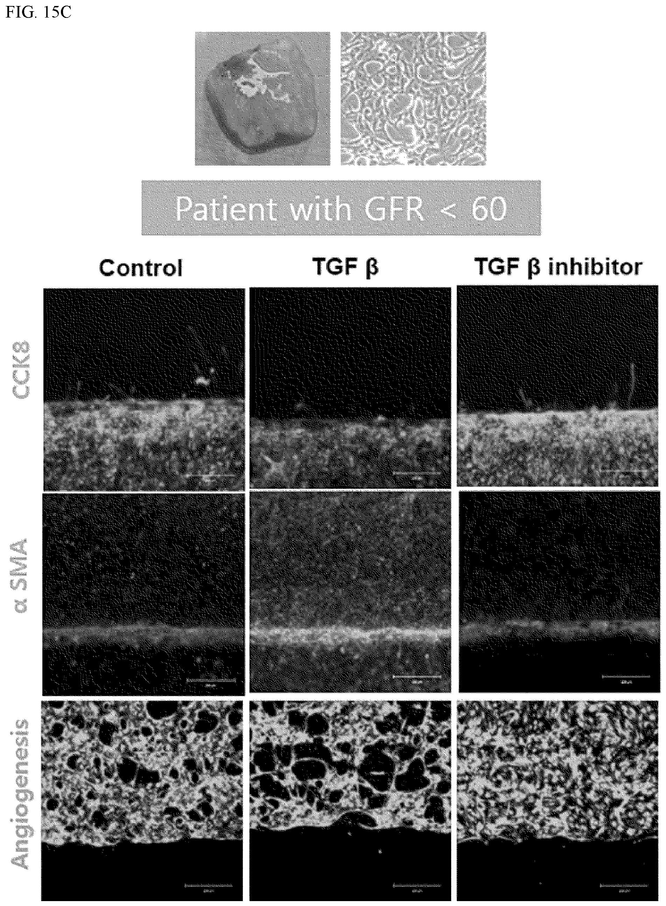

It is more reasonable to compare the kidney-on-a-chip of Example 3 to the pathological tissue of a patient from which kidney cells were obtained rather than comparing the kidney-on-a-chip to a laboratory animal model of renal fibrosis, so the evaluation indices for screening for renal fibrosis therapeutic agents were deduced by manufacturing a kidney-on-a-chip and performing comparative analysis in the same manner as in Experimental Example 1. At this time, the three patients with renal fibrosis who provided kidney tissue had a glomerular filtration rate (GFR) of more than 90 (Patient with GFR > 90 in FIG. 14), and 60 to 90 (Patient with GFR 60-90 in FIG. 14), and less than 60 (Patient with GFR < 60 in FIG. 14), respectively. In this experiment, three types of kidney-on-chips were manufactured: the control group (control in FIGS. 15 to 20) not treated with TGF-β, the TGF-β group (TGF β in FIGS. 15 to 20) treated with TGF-β in Experimental Example 1, and a TGF-β inhibition group (TGF β inhibitor in FIGS. 15 to 20) that was treated with TGF-β at 5 ng/ml and a TGF-β inhibitor at 10 µM and reacted for 24 hours.

### [Experimental Example 2-1] Confirmation of CCK8 and α-SMA as evaluation indices for screening for renal fibrosis therapeutic agents

The three types of kidney-on-chips manufactured above were treated with CCK8 antibody and α-SMA antibody, respectively, in the same manner as in Experimental Example 1, and comparative analysis of their fluorescence expression was performed. The results are as illustrated in FIGS. 15 and 16.

As illustrated in FIGS. 15 and 16, it has been confirmed that in all of the three types of renal fibrosis-on-chips manufactured using cells derived from kidney tissue of patients with renal fibrosis by the method, the expression level of CCK8 is decreased at the time of TGF-β stimulation and increased at the time of administration of TGF-β inhibitor but the expression level of α-SMA is increased at the time of TGF-β stimulation and decreased at the time of administration of TGF-β inhibitor in the renal tubular epithelial cell region of the renal tubule culture part, and thus the markers CCK8 and α-SMA can be evaluation indices for screening for renal fibrosis therapeutic agents.

### [Experimental Example 2-2] Confirmation of degree of angiogenesis as evaluation index for screening for renal fibrosis therapeutic agents

For the three types of kidney-on-chips of the control group, the TGF-β group, and the TGF-β inhibition group, vascular endothelial cells (GFP-HUVEC) present in the vascular endothelial cell region of the interstitium culture part were photographed using a confocal microscope (MICROSCOPE CONFOCAL LASER SCANING ZEISS LSM 710). Using the Imaged program, the obtained images were used to measure the length and diameter of thin blood vessels having a diameter of less than 50 µm and the length and diameter of thick blood vessels having a diameter of 50 µm or more, respectively. The result values measured for each of the control group, TGF-β group, and TGF-β inhibition group were compared to the values for the control group according to each index to comparatively analyze the length and diameter of thin blood vessels and the length and diameter of thick blood vessels, and the results are as illustrated in FIGS. 17 to 18.

As illustrated in FIGS. 17 to 18, in the three types of renal fibrosis-on-chips manufactured above, at the time of TGF-β stimulation, the total length of thick blood vessels (50 µm or more) in the vascular endothelial cell region of the interstitium culture part is decreased (FIG. 17A) and the diameter of thick blood vessels is also decreased (FIG. 17B) but the total length of thin blood vessels (less than 50 µm) is increased (FIG. 18A) and the diameter of thin blood vessels is decreased (FIG. 18B), but these changes are offset by a TGF-β inhibitor.

As a result, epithelial-mesenchymal transition (EMT) caused by TGF-β stimulation can be confirmed using the kidney-on-a-chip according to an embodiment of the present invention. It has been confirmed that the proliferation of vascular endothelial cells is more active and thick blood vessels are well formed particularly in the case of renal fibrosis-on-chips manufactured using cells derived from patients with reduced kidney function and more advanced fibrosis and vascular damage in kidney tissue (namely, patients having low GFR levels), and changes in vascular structure at the time of TGF-β stimulation are relatively minor in renal fibrosis-on-chips manufactured using cells derived from patients with severe kidney damage (namely, patients having low GFR levels) compared to renal fibrosis-on-chips manufactured using cells derived from patients with mild kidney damage (namely, patients having high GFR levels) (FIGS. 17 and 18). Through this, it has been indirectly confirmed that humoral factors that induce vascular proliferation are secreted from renal fibroblasts derived from tissues with severe kidney damage, and it has been found that these changes appear as fibro-intimal thickening and arteriolosclerosis in the patient's kidney tissue.

Through this, it has been confirmed that renal fibrosis therapeutic agents can be screened for by measuring CCK8 expression level, α-SMA expression level, and the degree of angiogenesis through measurement of blood vessel diameter, which are evaluation indices identified in Experimental Example 1 using the kidney-on-chips according to an embodiment of the present invention.

### [Experimental Example 3] Deduction of additional evaluation indices for screening for renal fibrosis therapeutic agents

It was confirmed that renal fibrosis therapeutic agents could be screened for using the kidney-on-chips according to an embodiment of the present invention through Experimental Examples 1 and 2, and additional evaluation indices were deduced in the following manner.

### [Experimental Example 3-1] Confirmation of KIM-1 as evaluation index for screening for renal fibrosis therapeutic agents

An experiment was performed in the same manner as in Experimental Example 2, but enzyme-linked immunosorbent assay (ELISA assay) was performed to examine whether kidney injury molecule-1 (KIM-1) could be used as an evaluation index.

Specifically, renal fibroblasts obtained from each of three patients having GFP > 90, GFP 60 to 90, and GFP < 60 and purchased vascular endothelial cells (GFP-HUVEC) and renal tubular epithelial cells (HK-2 cells) were co-cultured in the vascular endothelial cell medium in the same manner as in Experimental Example 1 to manufacture three types of kidney-on-chips. Next, the medium was collected for each of the control group, TGF-β group, and TGF-β inhibition group of each of the three types of kidney-on-chips, the medium of each group was diluted at an appropriate ratio, and then ELISA analysis was performed according to the instructions of the KIM-1 (human) ELISA kit (Enzo Life Sciences, Inc, # ADI-900-226-0001). Afterwards, the result values for each group were measured at 450 nm using ELISA READER (SPECTRAMAX PLUS384), each measured result value was compared to the value for the control group of each of the three types of kidney-on-chips to comparatively analyze the difference in expression level of KIM-1, and the results are as illustrated in FIG. 19.

As illustrated in FIG. 19, it has been confirmed that in the three types of renal fibrosis-on-chips, the expression level of KIM-1 in the culture solution (LONZA #CC-3162 EGM-2.) of vascular endothelial cells (GFP-HUVEC) used as a culture solution for co-culture of cells is increased at the time of TGF-β stimulation and these changes are offset when a TGF-β inhibitor is administered. In particular, as the kidney function of patients whose cells are used to manufacture each kidney-on-a-chip is decreased, the expression level of KIM-1 and the degree of changes caused by TGF-β stimulation or a TGF-β inhibitor are decreased. Through this, it has been confirmed that renal fibrosis therapeutic agents can be screened for by measuring the expression level of KIM-1 using the kidney-on-chips according to an embodiment of the present invention.

### [Experimental Example 3-2] Confirmation of VEGF as evaluation index for screening for renal fibrosis therapeutic agents

An experiment was performed in the same manner as in Experimental Example 3-1, but real-time PCR was performed to examine whether the vascular endothelial growth factor (VEGF) can be used as an evaluation index.

Specifically, for each of the control group, TGF-β group, and TGF-β inhibition group of each of the two types of kidney-on-chips manufactured from a patient with GFP > 90 and a patient with GFP 60 to 90 in Experimental Example 3-1, RNA was isolated from all of the renal tubular epithelial cells (HK-2 cells), fibroblasts, and vascular endothelial cells (GFP-HUVEC) co-cultured in each kidney-on-a-chip, cDNA was synthesized from this according to a method known in the art and mixed with VEGF primers and SYBER green, and the expression level of VEGF was measured using real-time PCR equipment (REAL TIME PCR SYSTEM, Applied Biosystems ViiA7). After measurement, the result values for each group measured using the equipment were compared to the values for the control group of each of the three types of kidney-on-chips to comparatively analyze the difference in expression level of VEGF, and the results are as illustrated in FIG. 20.

As illustrated in FIG. 20, it has been confirmed that in the two types of renal fibrosis-on-chips, the expression level of VEGF is increased at the time of TGF-β stimulation and decreased at the time of administration of TGF-β inhibitor. In particular, as the kidney function of patients whose cells are used to manufacture each kidney-on-a-chip is decreased (that is, as the GFP level decreases), the expression level of VEGF and the degree of changes caused by TGF-β stimulation or a TGF-β inhibitor decrease.

Through this, it has been confirmed that renal fibrosis therapeutic agents can be screened for by adopting the expression level of KIM-1 and the expression level of VEGF as evaluation indices and using the kidney-on-chips according to an embodiment of the present invention.

## Claims

1. A kidney-on-a-chip comprising:
a renal tubule culture part where renal tubular epithelial cells are cultured; and
an interstitium culture part where renal fibroblasts and vascular endothelial cells are cultured,
wherein the renal tubule culture part and the interstitium culture part are horizontally arranged, and
the renal tubular epithelial cells, renal fibroblasts, and vascular endothelial cells are co-cultured.

2. The kidney-on-a-chip according to claim 1,
wherein the interstitium culture part contains fibrin gel.

3. The kidney-on-a-chip according to claim 1,
wherein one or more cells selected from the group consisting of the renal tubular epithelial cells, renal fibroblasts, and vascular endothelial cells are human-derived cells.

4. The kidney-on-a-chip according to claim 1,
wherein one or more cells selected from the group consisting of the renal tubular epithelial cells, renal fibroblasts, and vascular endothelial cells are renal tubular epithelial cells derived from a patient with kidney disease.

5. The kidney-on-a-chip according to claim 1,
wherein one or more cells selected from the group consisting of the renal tubular epithelial cells, renal fibroblasts, and vascular endothelial cells are cells undergoing fluorescence staining.

6. The kidney-on-a-chip according to claim 1,
wherein the kidney-on-a-chip is a renal fibrosis-on-a-chip.

7. The kidney-on-a-chip according to claim 1,
wherein the kidney-on-a-chip comprises two or more interstitium culture parts, and
the renal tubule culture part is located between the two or more interstitium culture parts.

8. A method for manufacturing the kidney-on-a-chip according to any one of claims 1 to 7, the method comprising:
culturing renal tubular epithelial cells in the renal tubule culture part and culturing renal fibroblasts and vascular endothelial cells in the interstitium culture part.

9. The method for manufacturing a kidney-on-a-chip according to claim 8,
wherein the manufacturing method further comprising isolating one or more selected from the group consisting of renal tubular epithelial cells, renal fibroblasts, and vascular endothelial cells from human kidney tissue before the cell culturing step.

10. A co-culture device comprising:
a renal tubule culture part where renal tubular epithelial cells are cultured; and
an interstitium culture part where renal fibroblasts and vascular endothelial cells are cultured,
wherein the renal tubule culture part and the interstitium culture part are horizontally arranged, and
the renal tubular epithelial cells, renal fibroblasts, and vascular endothelial cells are co-cultured.

11. A method for screening for a renal fibrosis therapeutic agent, the method comprising:
(a) treating the kidney-on-a-chip according to any one of claims 1 to 7 with transforming growth factor-β (TGF-β);
(b) treating the kidney-on-a-chip with a test substance; and
(c) measuring a renal fibrosis evaluation index in the kidney-on-a-chip treated with the test substance.

12. The method for screening for a renal fibrosis therapeutic agent according to claim 11,
wherein one or more selected from the group consisting of the renal tubular epithelial cells, renal fibroblasts, and vascular endothelial cells in the kidney-on-a-chip are renal tubular epithelial cells derived from a patient with kidney disease.

13. The method for screening for a renal fibrosis therapeutic agent according to claim 11,
wherein step (c) includes comparing a renal fibrosis evaluation index before the kidney-on-a-chip is treated with a test substance to the renal fibrosis evaluation index after the kidney-on-a-chip is treated with the test substance or a renal fibrosis evaluation index when the kidney-on-a-chip is not treated with a test substance to the renal fibrosis evaluation index when the kidney-on-a-chip is treated with the test substance.

14. The method for screening for a renal fibrosis therapeutic agent according to claim 11,
wherein step (c) includes comparing a renal fibrosis evaluation index in a kidney-on-a-chip that is treated with a test substance to the renal fibrosis evaluation index in a kidney-on-a-chip that is not treated with the test substance or is not yet treated with the test substance.

15. The method for screening for a renal fibrosis therapeutic agent according to claim 11,
wherein step (c) includes measuring relative expression levels of one or more selected from the group consisting of cytokeratin 8 (CCK8) and α-smooth muscle actin (α-SMA) in the renal tubular epithelial cells.

16. The method for screening for a renal fibrosis therapeutic agent according to claim 15,
further comprising determining the test substance as a renal fibrosis therapeutic agent when an expression level of CCK8 after treatment with the test substance is increased or an expression level of α-SMA after treatment with the test substance is decreased compared to the expression level of CCK8 or the expression level of α-SMA without treatment with the test substance or before treatment with the test substance as a result of measuring the relative expression levels in step (c).

17. The method for screening for a renal fibrosis therapeutic agent according to claim 11,
wherein step (c) includes measuring degree of angiogenesis in the interstitium culture part where vascular endothelial cells are cultured,
wherein the degree of angiogenesis is sum of lengths of blood vessels having a thickness of less than 50 µm among blood vessels formed in the interstitium culture part.

18. The method for screening for a renal fibrosis therapeutic agent according to claim 15,
further comprising determining the test substance as a renal fibrosis therapeutic agent when the degree of angiogenesis after treatment with the test substance is decreased compared to the degree of angiogenesis without treatment with the test substance or before treatment with the test substance as a result of measuring the degree of angiogenesis in step (c).

19. The method for screening for a renal fibrosis therapeutic agent according to claim 11,
wherein step (c) includes measuring a relative expression level of cluster of differentiation 31 (CD31) in the vascular endothelial cells.

20. The method for screening for a renal fibrosis therapeutic agent according to claim 19,
further comprising determining the test substance as a renal fibrosis therapeutic agent when the relative expression level of CD31 after treatment with the test substance is decreased compared to the relative expression level of CD31 without treatment with the test substance or before treatment with the test substance as a result of measuring the relative expression level in step (c).

21. The method for screening for a renal fibrosis therapeutic agent according to claim 11,
wherein step (c) includes measuring relative expression levels of one or more selected from the group consisting of kidney injury molecule-1 (KIM-1) and vascular endothelial growth factor (VEGF) in a culture solution in the kidney-on-a-chip.

22. The method for screening for a renal fibrosis therapeutic agent according to claim 21,
further comprising determining the test substance as a renal fibrosis therapeutic agent when expression levels of one or more selected from the group consisting of KIM-1 and VEGF after treatment with the test substance are decreased compared to the expression levels without treatment with the test substance or before treatment with the test substance as a result of measuring the relative expression levels in step (c).
